(19) Europäisches Patentamt / European Patent Office / Office européen des brevets

(11) **EP 1 611 079 B1**

(12) **EUROPÄISCHE PATENTSCHRIFT**

(45) Veröffentlichungstag und Bekanntmachung des Hinweises auf die Patenterteilung:
**23.05.2007 Patentblatt 2007/21**

(21) Anmeldenummer: **04722167.6**

(22) Anmeldetag: **20.03.2004**

(51) Int Cl.:
**C07C 57/055** (2006.01)

(86) Internationale Anmeldenummer:
**PCT/EP2004/002934**

(87) Internationale Veröffentlichungsnummer:
**WO 2004/085370 (07.10.2004 Gazette 2004/41)**

(54) **VERFAHREN DER HETEROGEN KATALYSIERTEN PARTIELLEN GASPHASENOXIDATION VON ACROLEIN ZU ACRYLS URE**

METHOD FOR THE HETEROGENEOUSLY CATALYSED PARTIAL GAS PHASE OXIDATION OF ACROLEIN TO FORM ACRYLIC ACID

PROCEDE D'OXYDATION PARTIELLE EN PHASE GAZEUSE D'ACROLEINE EN ACIDE ACRYLIQUE PAR CATALYSE HETEROGENE

(84) Benannte Vertragsstaaten:
**AT BE BG CH CY CZ DE DK EE ES FI FR GB GR HU IE IT LI LU MC NL PL PT RO SE SI SK TR**

(30) Priorität: **25.03.2003 DE 10313214**
**05.06.2003 US 475792 P**

(43) Veröffentlichungstag der Anmeldung:
**04.01.2006 Patentblatt 2006/01**

(73) Patentinhaber: **BASF Aktiengesellschaft**
**67056 Ludwigshafen (DE)**

(72) Erfinder:
- DIETERLE, Martin
  68167 Mannheim (DE)
- PETZOLDT, Jochen
  68163 Mannheim (DE)
- ARNOLD, Heiko
  Nanjing 210014 (CN)
- RUPPEL, Wilhelm
  68163 Mannheim (DE)
- MÜLLER-ENGEL, Klaus, Joachim
  76297 Stutensee (DE)

(56) Entgegenhaltungen:
EP-A- 1 055 662          WO-A-00/53559

**Beschreibung**

Vorliegende Erfindung betrifft ein Verfahren der heterogen katalysierten partiellen Gasphasenoxidation von Acrolein zu Acrylsäure, bei dem man ein Acrolein, molekularen Sauerstoff und wenigstens ein Inertgas enthaltendes Reaktionsgasausgangsgemisch, das den molekularen Sauerstoff und das Acrolein in einem molaren Verhältnis $O_2$ : $C_3H_4O \geq 0,5$ enthält, in einer Reaktionsstufe mit der Maßgabe über eine Festbettkatalysatorschüttung, deren Aktivmasse wenigstens ein die Elemente Mo und V enthaltendes Multimetalloxid ist, führt, dass

- die Festbettkatalysatorschüttung in zwei räumlich aufeinanderfolgenden Temperaturzonen A, B angeordnet ist,

- sowohl die Temperatur der Temperaturzone A als auch die Temperatur der Temperaturzone B eine Temperatur im Bereich von 230 bis 320°C ist,

- die Festbettkatalysatorschüttung aus wenigstens zwei räumlich aufeinanderfolgenden Festbettkatalysatorschüttungszonen besteht, wobei die volumenspezifische Aktivität innerhalb einer Festbettkatalysatorschüttungszone im wesentlichen konstant ist und in Strömungsrichtung des Reaktionsgasgemisches beim Übergang von einer Festbettkatalysatorschüttungszone in eine andere Festbettkatalysatorschüttungszone sprunghaft zunimmt,

- sich die Temperaturzone A bis zu einem Umsatz des Acroleins von 45 bis 85 mol-% erstreckt,

- bei einmaligem Durchgang des Reaktionsgasausgangsgemisches durch die gesamte Festbettkatalysatorschüttung der Acroleinumsatz ≥90 mol-% und die Selektivität der Acrylsäurebildung, bezogen auf umgesetztes Acrolein, ≥90 mol-% beträgt,

- die zeitliche Abfolge, in der das Reaktionsgasgemisch die Temperaturzonen A, B durchströmt, der alphabetischen Abfolge der Temperaturzonen entspricht,

- die Belastung der Festbettkatalysatorschüttung mit dem im Reaktionsgasausgangsgemisch enthaltenen Acrolein ≥70 NI Acrolein/l Festbettkatalysatorschüt tung • h beträgt, und

- die Differenz $T^{maxA}$ - $T^{maxB}$, gebildet aus der höchsten Temperatur $T^{maxA}$, die das Reaktionsgasgemisch innerhalb der Temperaturzone A aufweist, und der höchsten Temperatur $T^{maxB}$, die das Reaktionsgasgemisch innerhalb der Temperaturzone B aufweist, ≥0°C beträgt.

Das vorgenannte Verfahren der heterogen katalytisierten partiellen Gasphasenoxidation von Acrolein zu Acrylsäure ist z.B. aus der DE-A 19910508 prinzipiell bekannt und insbesondere als zweite Oxidationsstufe bei der Herstellung von Acrylsäure durch zweistufige katalytische Gasphasenoxidation ausgehend von Propen von Bedeutung. Acrylsäure ist ein bedeutendes Monomeres, das als solches oder in Form seiner Alkylester zur Erzeugung von z.B. als Klebstoffen geeigneten Polymerisaten Verwendung findet.

Neben molekularem Sauerstoff und den Reaktanden enthält das Reaktionsgasausgangsgemisch u.a. deshalb Inertgas, um das Reaktionsgas außerhalb des Explosionsbereiches zu halten.

Eine Zielsetzung einer solchen heterogen katalysierten partiellen Gasphasenoxidation von Acrolein zu Acrylsäure besteht darin, bei einmaligem Durchgang des Reaktionsgasgemisches durch die Reaktionsstufe unter ansonsten vorgegebenen Randbedingungen eine möglichst hohe Selektivität $S^{AA}$ an Acrylsäure zu erzielen (das ist die Molzahl von zu Acrylsäure umgesetztem Acrolein, bezogen auf die Molzahl an umgesetztem Acrolein).

Eine weitere Zielsetzung besteht darin, bei einmaligem Durchgang des Reaktionsgasgemisches durch die Reaktionsstufe unter ansonsten vorgegebenen Randbedingungen einen möglichst hohen Umsatz $U^{AC}$ an Acrolein zu erzielen (das ist die Molzahl von umgesetztem Acrolein, bezogen auf die Molzahl an eingesetztem Acrolein).

Ist bei hohem $U^{AC}$ die $S^{AA}$ hoch, entspricht dies einer hohen Ausbeute $A^{AA}$ an Acrylsäure ($A^{AA}$ ist das Produkt $U^{AC} \cdot S^{AA}$; d.h., die Molzahl von zu Acrylsäure umgesetztem Acrolein, bezogen auf die Molzahl an eingesetztem Acrolein).

Eine weitere Zielsetzung einer solchen heterogen katalysierten partiellen Gasphasenoxidation von Acrolein zu Acrylsäure besteht darin, eine möglichst hohe Raum-Zeit-Ausbeute ($RZA^{AA}$) an Acrylsäure zu erzielen (das ist bei einer kontinuierlichen Verfahrensweise die je Stunde und Volumen der verwendeten Festbettkatalysatorschüttung in Litern erzeugte Gesamtmenge an Acrylsäure).

Bei gleich bleibender gegebener Ausbeute $A^{AA}$ ist die Raum-Zeit-Ausbeute um so grö-ßer, je größer die Belastung der Festbettkatalysatorschüttung der Reaktionsstufe mit Acrolein ist (darunter wird die Menge an Acrolein in Normlitem (= NI; das Volumen in Litern, das die entsprechende Acroleinmenge bei Normalbedingungen, d.h., bei 25°C und 1 bar, einnehmen würde) verstanden, die als Bestandteil des Reaktionsgasausgangsgemisches pro Stunde durch einen Liter an Festbettkatalysatorschüttung geführt wird).

Die DE-A 19910508 und die WO 00/53559 lehren zwar, dass die vorgenannten Ziele mittels des eingangs beschriebenen Verfahrens der heterogen katalysierten partiellen Gasphasenoxidation von Acrolein zu Acrylsäure prinzipiell zu erreichen sind. Dies ist insofern von Vorteil, als sich eine geringere volumenspezifische Aktivität innerhalb einer Festbettkatalysatorschüttung bzw. Festbettkatalysatorschüttungszone z.B. dadurch erzielen lässt, dass man die eigentlichen, Aktivmasse tragenden, Katalysatorformkörper mit an Aktivmasse freien inerten Verdünnungsformkörpern verdünnt wodurch die Festbettkatalysatorschüttung insgesamt kostengünstiger wird.

Nachteilig an den Lehren der DE-A 19910508 sowie der WO 00/53559 ist jedoch, dass sie kein entsprechendes Ausführungsbeispiel aufweisen und somit die konkrete Ausgestaltung einer solchen Verfahrensweise offen lassen.

In ähnlicher Weise lehrt auch die EP-A 1106598 ein Verfahren der heterogen katalysierten partiellen Gasphasenoxidation von Acrolein zu Acrylsäure, bei dem die Festbettkatalysatorschüttung der Reaktionsstufe aus mehreren räumlich aufeinander folgenden Festbettkatalysatorschüttungszonen besteht, deren volumenspezifische Aktivität innerhalb einer Festbettkatalysatorschüttungszone im wesentlichen konstant ist und in Strömungsrichtung des Reaktionsgasgemisches beim Übergang von einer Festbettkatalysatorschüttungszone in eine andere Festbettkatalysatorschüttungszone sprunghaft zunimmt und in mehreren Temperaturzonen angeordnet sein kann.

Gemäß der Lehre der EP-A 1106598 ist es dabei zweckmäßig, wenn Festbettkatalysatorschüttungszonen und Temperaturzonen räumlich zusammenfallen.

Eingehende Untersuchungen seitens der Anmelderin haben jedoch ergeben, dass ein räumliches Zusammenfallen von Festbettkatalysatorschüttungszonen und Temperaturzonen für ein optimales Erreichen der verschiedenen angesprochenen Zielsetzungen in der Regel nicht förderlich ist.

Dies ist u.a. darauf zurückzuführen, dass der Übergang von der Temperaturzone A in die Temperaturzone B und der Übergang von einer Festbettkatalysatorschüttungszone in eine andere Festbettkatalysatorschüttungszone entweder als im wesentlichen gleich wirkende oder als im wesentlichen entgegengesetzt wirkende Maßnahmen ergriffen werden.

Werden sie als im wesentlichen gleich wirkende Maßnahmen ergriffen (z.B. Übergang von einer kälteren Temperaturzone A in eine heißere Temperaturzone B und Übergang von einer Festbettkatalysatorschüttungszone mit niederer volumenspezifischer Aktivität in eine Festbettkatalysatorschüttungszone mit höherer volumenspezifischer Aktivität; beide Maßnahmen verfolgen den Zweck, die Reaktion zu fördern), so schießt die erzielte Gesamtwirkung im Übergangsbereich häufig über das angestrebte Ziel hinaus und es resultiert z.B. eine Minderung der $S^{AA}$.

Werden sie als im wesentlichen entgegengesetzt wirkende Maßnahmen ergriffen (z.B. Übergang von einer heißeren Temperaturzone A in eine kältere Temperaturzone B und Übergang von einer Festbettkatalysatorschüttungszone mit niederer volumenspezifischer Aktivität in eine Festbettkatalysatorschüttungszone mit höherer volumenspezifischer Aktivität; die erste Maßnahme verfolgt den Zweck, die Reaktion zu mindern, die zweite Maßnahme verfolgt den Zweck, die Reaktion zu fördern), so neutralisieren sich die Einzelwirkungen im Übergangsbereich häufig und es resultiert ein geminderter $U^{AC}$.

Die Aufgabe der vorliegenden Erfindung bestand daher darin, ein Verfahren der heterogen katalysierten partiellen Gasphasenoxidation von Acrolein zu Acrylsäure in einer Mehrzonenanordnung zur Verfügung zu stellen, das die Nachteile der Verfahren des Standes der Technik nicht ausweist.

Demgemäss wurde ein Verfahren der heterogen katalysierten partiellen Gasphasenoxidation von Acrolein zu Acrylsäure, bei dem man ein Acrolein, molekularen Sauerstoff und wenigstens ein Inertgas enthaltendes Reaktionsgasausgangsgemisch, das den molekularen Sauerstoff und das Acrolein in einem molaren Verhältnis $O_2 : C_3H_4O \geq 0{,}5$ enthält, in einer Reaktionsstufe mit der Maßgabe über eine Festbettkatalysatorschüttung, deren Aktivmasse wenigstens ein die Elemente Mo und V enthaltendes Multimetalloxid ist, führt, dass

-    die Festbettkatalysatorschüttung in zwei räumlich aufeinanderfolgenden Temperaturzonen A, B angeordnet ist,

-    sowohl die Temperatur der Temperaturzone A als auch die Temperatur der Temperaturzone B eine Temperatur im Bereich von 230 bis 320˚C ist,

-    die Festbettkatalysatorschüttung aus wenigstens zwei räumlich aufeinanderfolgenden Festbettkatalysatorschüttungszonen besteht, wobei die volumenspezifische Aktivität innerhalb einer Festbettkatalysatorschüttungszone im wesentlichen konstant ist und in Strömungsrichtung des Reaktionsgasgemisches beim Übergang von einer Festbettkatalysatorschüttungszone in eine andere Festbettkatalysatorschüttungszone sprunghaft zunimmt,

-    sich die Temperaturzone A bis zu einem Umsatz des Acroleins von 45 bis 85 mol-% erstreckt,

-    bei einmaligem Durchgang des Reaktionsgasgemisches durch die gesamte Festbettkatalysatorschüttung der Acroleinumsatz ≥90 mol-% und die Selektivität der Acrylsäurebildung, bezogen auf umgesetztes Acrolein, ≥90 mol-% beträgt,

- die zeitliche Abfolge, in der das Reaktionsgasgemisch die Temperaturzonen A, B durchströmt, der alphabetischen Abfolge der Temperaturzonen entspricht,

- die Belastung der Festbettkatalysatorschüttung mit dem im Reaktionsgasausgangsgemisch enthaltenen Acrolein ≥70 Nl Acrolein/l Festbettkatalysatorschüttung • h beträgt, und

- die Differenz $T^{maxA}$ - $T^{maxB}$, gebildet aus der höchsten Temperatur $T^{maxA}$, die das Reaktionsgasgemisch innerhalb der Temperaturzone A aufweist, und der höchsten Temperatur $T^{maxB}$, die das Reaktionsgasgemisch innerhalb der Temperaturzone B aufweist, ≥0˚C beträgt,

gefunden, das dadurch gekennzeichnet ist,
dass der Übergang von der Temperaturzone A in die Temperaturzone B in der Festbettkatalysatorschüttung (räumlich) nicht mit einem Übergang von einer Festbettkatalysatorschüttungszone in eine andere Festbettkatalysatorschüttungszone zusammenfällt.

Unter der Temperatur einer Temperaturzone wird in dieser Schrift die Temperatur des in der Temperaturzone befindlichen Teils der Festbettkatalysatorschüttung bei Ausübung des erfindungsgemäßen Verfahrens, jedoch in Abwesenheit einer chemischen Reaktion verstanden. Ist diese Temperatur innerhalb der Temperaturzone nicht konstant, so meint der Begriff Temperatur einer Temperaturzone hier den (Zahlen)mittelwert der Temperatur der Festbettkatalysatorschüttung längs der Reaktionszone. Wesentlich ist dabei, dass die Temperierung der einzelnen Temperaturzonen im wesentlichen unabhängig voneinander erfolgt.

Da die heterogen katalysierte partielle Gasphasenoxidation von Acrolein zu Acrylsäure eine ausgeprägt exotherme Reaktion ist, ist die Temperatur des Reaktionsgasgemisches beim reaktiven Durchgang durch die Festbettkatalysatorschüttung in der Regel von der Temperatur einer Temperaturzone verschieden. Sie liegt normalerweise oberhalb der Temperatur der Temperaturzone und durchläuft innerhalb einer Temperaturzone in der Regel ein Maximum (Heißpunktmaximum) oder fällt von einem Maximalwert ausgehend ab.

In der Regel wird beim erfindungsgemäßen Verfahren die Differenz $T^{maxA}$ - $T^{maxB}$ nicht mehr als 75˚C betragen. Erfindungsgemäß bevorzugt beträgt $T^{maxA}$ - $T^{maxB}$ ≥3˚C und ≤ 60˚C. Ganz besonders bevorzugt beträgt $T^{maxA}$ - $T^{maxB}$ beim erfindungsgemäßen Verfahren ≥5˚C und ≤40˚C.

Die erfindungsgemäß geforderten Differenzen $T^{maxA}$ - $T^{maxB}$ stellen sich bei der Ausübung des erfindungsgemäßen Verfahrens im Fall von eher niederen (≥70 Nl/l • h und < 150 Nl/l • h) Acroleinbelastungen der Festbettkatalysatorschüttung normalerweise dann ein, wenn einerseits sowohl die Temperatur der Reaktionszone A als auch die Temperatur der Reaktionszone B im Bereich von 230 bis 320˚C liegt und andererseits die Differenz zwischen der Temperatur der Reaktionszone B ($T_B$) und der Temperatur der Reaktionszone A ($T_A$), d.h., $T_B$ - $T_A$, ≤0˚C und ≥-20˚C oder ≥-10˚C; bzw. ≤0˚C und ≥-5˚C, oder häufig ≤0˚C und ≥-3˚C beträgt.

Bei Ausübung des erfindungsgemäßen Verfahrens unter erhöhten Acroleinbelastungen (≥150 Nl/l • h und ≤300 Nl/l • h bzw. ≤600 Nl/l • h) stellen sich die erfindungsgemäß geforderten Differenzen $T^{maxA}$ - $T^{maxB}$ normalerweise dann ein, wenn einerseits sowohl die Temperatur der Reaktionszone A als auch die Temperatur der Reaktionszone B im Bereich von 230 bis 320˚C liegt und $T_B$ - $T_A$ > 0˚C und ≤40˚C, oder ≥5˚C und ≤35˚C bzw. 30˚C, oder ≥10˚C und ≤25˚C bzw. 20˚C, oder 15˚C beträgt.

Die vorgenannte Aussage betreffend die Temperaturdifferenzen $T_B$ - $T_A$ gilt regelmäßig auch dann, wenn die Temperatur der Reaktionszone A im bevorzugten Bereich von 250 bis 300˚C bzw. im besonders bevorzugten Bereich von 260 bis 280˚C liegt.

Die Acroleinbelastung der Festbettkatalysatorschüttung kann somit beim erfindungsgemäßen Verfahren z.B. ≥70 Nl/l • h bzw. ≥90 Nl/l • h und ≤300 Nl/l • h, oder ≥ 110 Nl/l • h und ≤280 Nl/l • h, oder ≥130 Nl/l • h und ≤260 Nl/l • h, oder ≥150 Nl/l • h und ≥240 Nl/l • h, oder ≥170 Nl/l • h und ≤220 Nl/l • h, oder ≥190 Nl/l • h und ≤ 200 Nl/l • h betragen.

Erfindungsgemäß bevorzugt erstreckt sich die Temperaturzone A bis zu einem Umsatz des Acroleins von 50 bis 85 mol-% bzw. 60 bis 85 mol-%.

Der Arbeitsdruck kann beim erfindungsgemäßen Verfahren sowohl unterhalb von Normaldruck (z.B. bis zu 0,5 bar) als auch oberhalb von Normaldruck liegen. Typischerweise wird der Arbeitsdruck bei Werten von 1 bis 5 bar, häufig 1 bis 3 bar liegen. Normalerweise wird der Reaktionsdruck 100 bar nicht überschreiten.

Das molare Verhältnis von $O_2$: Acrolein im Reaktionsgasausgangsgemisch muß erfindungsgemäß ≥0,5 betragen. Häufig wird es bei Werten ≥1 liegen. Üblicherweise wird dieses Verhältnis bei Werten ≤3 liegen. Häufig beträgt das molare Verhältnis von $O_2$: Acrolein im Reaktionsgasausgangsgemisch erfindungsgemäß 1 bis 2 bzw. 1 bis 1,5.

In der Regel kann der auf den einfachen Durchgang bezogene Acroleinumsatz beim erfindungsgemäßen Verfahren ≥92 mol-%, oder ≥94 mol-%, oder ≥96 mol-%, oder ≥98 mol-% und häufig sogar ≥99 mol-% betragen. Die Selektivität der Acrylsäurebildung wird dabei regelmäßig ≥92 mol-%, bzw. ≥94 mol-%, häufig ≥95 mol-% oder ≥96 mol-% bzw. ≥97 mol-% betragen.

Als Katalysatoren für die Festbettkatalysatorschüttung der erfindungsgemäßen gasphasenkatalytischen Acroleinoxidation kommen alle diejenigen in Betracht, deren Aktivmasse wenigstens ein Mo und V enthaltendes Multimetalloxid ist. Solchermaßen geeignete Mulitmetalloxidkatalysatoren können beispielsweise der US-A 3 775 474, der US-A 3954855, der US-A 3893951 und der US-A 4339355 entnommen werden. Ferner eignen sich in besonderer Weise die Multimetalloxidmassen der EP-A 427508, der DE-A 2909671, der DE-C 3151805, der DE-AS 2626887, der DE-A 4302991, der EP-A 700893, der EP-A 714700 und der DE-A 19736105 sowie der DE-A 10046928.

Geeignet sind in diesem Zusammenhang auch die beispielhaften Ausführungsformen der EP-A 714700 sowie der DE-A 19736105.

Eine Vielzahl der für die Festbettkatalysatorschüttung geeigneten Multimetalloxidaktivmassen, z.B. diejenigen der DE-A 19815281, lässt sich unter der allgemeinen Formel IV

$$Mo_{12}V_aX^1_bX^2_cX^3_dX^4_eX^5_fX^6_gO_n \qquad \text{(IV)},$$

in der die Variablen folgende Bedeutung haben:

$X^1$ = W, Nb, Ta, Cr und/oder Ce,
$X^2$ = Cu, Ni, Co, Fe, Mn und/oder Zn,
$X^3$ = Sb und/oder Bi,
$X^4$ = eines oder mehrere Alkalimetalle,
$X^5$ = eines oder mehrere Erdalkalimetalle,
$X^6$ = Si, Al, Ti und/oder Zr,

a = 1 bis 6,
b = 0,2 bis 4,
c = 0,5 bis 18,
d = 0 bis 40,
e = 0 bis 2,
f = 0 bis 4,
g = 0 bis 40 und
n = eine Zahl, die durch die Wertigkeit und Häufigkeit der von Sauerstoff verschiedenen Elemente in IV bestimmt wird,

subsummieren.

Erfindungsgemäß bevorzugte Ausführungsformen innerhalb der aktiven Multimetalloxide IV sind jene, die von den nachfolgenden Bedeutungen der Variablen der allgemeinen Formel IV erfasst werden:

$X^1$ = W, Nb, und/oder Cr,
$X^2$ = Cu, Ni, Co, und/oder Fe,
$X^3$ = Sb,
$X^4$ = Na und/oder K,
$X^5$ = Ca, Sr und/oder Ba,
$X^6$ = Si, Al, und/oder Ti,

a = 1,5 bis 5,
b = 0,5 bis 2,
c = 0,5 bis 3,
d = 0 bis 2,
e = 0 bis 0,2,
f = 0 bis 1 und
n = eine Zahl, die durch die Wertigkeit und Häufigkeit der von Sauerstoff verschiedenen Elemente in IV bestimmt wird.

Erfindungsgemäß ganz besonders bevorzugte Multimetalloxide IV sind jedoch jene der allgemeinen Formel V

$$Mo_{12}V_{a'}Y^1_{b'}Y^2_{c'}Y^5_{f'}Y^6_{g'}O_n \qquad \text{(V)}$$

mit

$Y^1$ = W und/oder Nb,

$Y^2$ = Cu und/oder Ni,
$Y^5$ = Ca und/oder Sr,
$Y^6$ = Si und/oder Al,

a' = 2 bis 4,
b' = 1 bis 1,5,
c' = 1 bis 3,
f' = 0 bis 0,5
g' = 0 bis 8 und
n' = eine Zahl, die durch die Wertigkeit und Häufigkeit der von Sauerstoff ververschiedenen Elementen in V bestimmt wird.

Die erfindungsgemäß geeigneten Multimetalloxidaktivmassen (IV) sind in an sich bekannter, z.B. in der DE-A 4335973 oder in der EP-A 714700 offenbarter, Weise erhältlich.

Prinzipiell können für die Katalysatoren der Festbettkatalysatorschüttung geeignete Multimetalloxidaktivmassen, insbesondere solche der allgemeinen Formel IV, in einfacher Weise dadurch hergestellt werden, dass man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 350 bis 600˚C calciniert. Die Calcination kann sowohl unter Inertgas als auch unter einer oxidativen Atmosphäre wie z.B. Luft (Gemisch aus Inertgas und Sauerstoff) sowie auch unter reduzierender Atmosphäre (z.B. Gemische aus Inertgas und reduzierenden Gasen wie $H_2$, $NH_3$, CO, Methan und/oder Acrolein oder die genannten reduzierend wirkenden Gase für sich) durchgeführt werden. Die Calcinationsdauer kann einige Minuten bis einige Stunden betragen und nimmt üblicherweise mit der Temperatur ab. Als Quellen für die elementaren Konstituenten der Multimetalloxidaktivmassen IV kommen solche Verbindungen in Betracht, bei denen es sich bereits um Oxide handelt und/oder um solche Verbindungen, die durch Erhitzen, wenigstens in Anwesenheit von Sauerstoff, in Oxide überführbar sind.

Das innige Vermischen der Ausgangsverbindungen zur Herstellung von Multimetalloxidmassen IV kann in trockener oder in nasser Form erfolgen. Erfolgt es in trockener Form, so werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form.

Üblicherweise werden dabei die Ausgangsverbindungen in Form einer wässrigen Lösung und/oder Suspension miteinander vermischt. Besonders innige Trockengemische werden beim beschriebenen Mischverfahren dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene wässrige Masse getrocknet, wobei der Trocknungsprozess vorzugsweise durch Sprühtrocknung der wässrigen Mischung mit Austrittstemperaturen von 100 bis 150˚C erfolgt.

Die resultierenden Multimetalloxidmassen, insbesondere jene der allgemeinen Formel IV, werden in der Regel nicht in Pulverform sondern zu bestimmten Katalysatorgeometrien geformt in der Festbettkatalysatorschüttung eingesetzt, wobei die Formgebung vor oder nach der abschließenden Calcination erfolgen kann. Beispielsweise können aus der Pulverform der Aktivmasse oder ihrer uncalcinierten Vorläufermasse durch Verdichten zur gewünschten Katalysatorgeometrie (z.B. durch Tablettieren, Extrudieren oder Strangpressen) Vollkatalysatoren hergestellt werden, wobei gegebenenfalls Hilfsmittel wie z.B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können. Geeignete Vollkatalysatorgeometrien sind z.B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Selbstverständlich kann der Vollkatalysator auch Kugelgeometrie aufweisen, wobei der Kugeldurchmesser 2 bis 10 mm betragen kann.

Selbstverständlich kann die Formgebung der pulverförmigen Aktivmasse oder ihrer pulverförmigen, noch nicht calcinierten, Vorläufermasse auch durch Aufbringen auf vorgeformte inerte Katalysatorträger erfolgen. Die Beschichtung der Trägerkörper zur Herstellung der Schalenkatalysatoren wird in der Regel in einem geeigneten drehbaren Behälter ausgeführt, wie es z.B. aus der DE-A 2909671, der EP-A 293859 oder aus der EP-A 714700 bekannt ist.

Zweckmäßigerweise wird zur Beschichtung der Trägerkörper die aufzubringende Pulvermasse befeuchtet und nach dem Aufbringen, z.B. mittels heißer Luft, wieder getrocknet. Die Schichtdicke der auf den Trägerkörper aufgebrachten Pulvermasse wird zweckmäßigerweise im Bereich 10 bis 1000 $\mu$m, bevorzugt im Bereich 50 bis 500 $\mu$m und besonders bevorzugt im Bereich 150 bis 250 $\mu$m liegend, gewählt.

Als Trägermaterialien können dabei übliche poröse oder unporöse Aluminiumoxide, Siliciumdioxid, Thoriumdioxid, Zirkondioxid, Siliciumcarbid oder Silikate wie Magnesium- oder Aluminiumsilikat verwendet werden. Die Trägerkörper können regelmäßig oder unregelmäßig geformt sein, wobei regelmäßig geformte Trägerkörper mit deutlich ausgebildeter Oberflächenrauhigkeit, z.B. Kugeln oder Hohlzylinder mit Splittauflage, bevorzugt werden. Geeignet ist die Verwendung von im wesentlichen unporösen, oberflächenrauhen, kugelförmigen Trägem aus Steatit (z.B. Steatit C220 der Fa. CeramTec), deren Durchmesser 1 bis 8 mm, bevorzugt 4 bis 5 mm beträgt. Geeignet ist aber auch die Verwendung von Zylindern als Trägerkörper, deren Länge 2 bis 10 mm und deren Außendurchmesser 4 bis 10 mm beträgt. Im Fall von Ringen als Trägerkörper liegt die Wanddicke darüber hinaus üblicherweise bei 1 bis 4 mm. Bevorzugt zu verwendende ringförmige Trägerkörper besitzen eine Länge von 2 bis 6 mm, einen Außendurchmesser von 4 bis 8 mm und eine Wanddicke von 1 bis 2 mm. Geeignet sind vor allem auch Ringe der Geometrie 7 mm x 3 mm x 4 mm (Außendurchmesser x Länge x Innendurchmesser) als Trägerkörper. Die Feinheit der auf die Oberfläche des Trägerkörpers aufzubringenden katalytisch aktiven Oxidmassen wird selbstredend an die gewünschte Schalendicke angepasst (vgl. EP-A 714 700).

Günstige für die Katalysatoren der Festbettkatalysatorschüttung zu verwendende Multimetalloxidaktivmassen sind ferner Massen der allgemeinen Formel VI,

$$[D]_p[E]_q \qquad (VI),$$

in der die Variablen folgende Bedeutung haben:

$D = $ $Mo_{12}V_{a''}Z^1_{b''}Z^2_{c''}Z^3_{d''}Z^4_{e''}Z^5_{f''}Z^6_{g''}O_{x''}$,
$E = $ $Z^7_{12}Cu_{h''}H_{i''}O_{y''}$,
$Z^1 = $ W, Nb, Ta, Cr und/oder Ce,
$Z^2 = $ Cu, Ni, Co, Fe, Mn und/oder Zn,
$Z^3 = $ Sb und/oder Bi,
$Z^4 = $ Li, Na, K, Rb, Cs und/oder H
$Z^5 = $ Mg, Ca, Sr und/oder Ba,
$Z^6 = $ Si, Al, Ti und/oder Zr,
$Z^7 = $ Mo, W, V, Nb und/oder Ta, vorzugsweise Mo und/oder W

$a'' = $ 1 bis 8,
$b'' = $ 0,2 bis 5,
$c'' = $ 0 bis 23,
$d'' = $ 0 bis 50,
$e'' = $ 0 bis 2,
$f'' = $ 0 bis 5,
$g'' = $ 0 bis 50,
$h'' = $ 4 bis 30,
$i'' = $ 0 bis 20 und
$x'', y'' = $ Zahlen, die durch die Wertigkeit und Häufigkeit der von Sauerstoff verschiedenen Element in VI bestimmt werden und
$p, q = $ von Null verschiedene Zahlen, deren Verhältnis p/q 160:1 bis 1:1 beträgt,

und die dadurch erhältlich sind, dass man eine Multimetalloxidmasse E

$$Z^7_{12}CU_{h''}H_{i''}O_{y''} \qquad (E),$$

in feinteiliger Form getrennt vorbildet (Ausgangsmasse 1) und anschließend die vorgebildete feste Ausgangsmasse 1 in eine wässrige Lösung, eine wässrige Suspension oder in ein feinteiliges Trockengemisch von Quellen der Elemente Mo, V, $Z^1$, $Z^2$, $Z^3$, $Z^4$, $Z^5$, $Z^6$, die die vorgenannten Elemente in der Stöchiometrie D

$$Mo^{12}V_{a''}Z^1_{b''}Z^2_{c''}Z^3_{d''}Z^4_{e''}Z^5_{f''}Z^6_{g''} \qquad (D),$$

enthält (Ausgangsmasse 2), im gewünschten Mengenverhältnis p:q einarbeitet, die dabei gegebenenfalls resultierende wässrige Mischung trocknet, und die so gegebene trockene Vorläufermasse vor oder nach ihrer Trocknung zur gewünschten Katalysatorgeometrie bei Temperaturen von 250 bis 600˚C calciniert.

Bevorzugt sind die Multimetalloxidmassen VI, bei denen die Einarbeitung der vorgebildeten festen Ausgangsmasse 1 in eine wässrige Ausgangsmasse 2 bei einer Temperatur < 70˚C erfolgt. Eine detaillierte Beschreibung der Herstellung von Multimetalloxidmassen VI-Katalysatoren enthalten z.B. die EP-A 668104, die DE-A 19736105, die DE-A 10046928, die DE-A 19740493 und die DE-A 19528646.

Hinsichtlich der Formgebung gilt bezüglich Multimetalloxidmassen VI-Katalysatoren das bei den Multimetalloxidmassen IV-Katalysatoren Gesagte.

Darüber hinaus eignen sich als für die Katalysatoren der Festbettkatalysatorschüttung geeignete Multimetalloxidmassen jene der DE-A 19815281, insbesondere alle beispielhaften Ausführungsformen aus dieser Schrift. Hinsichtlich der Formgebung gilt das vorstehend Gesagte.

Für die Festbettkatalysatorschüttung des erfindungsgemäßen Verfahrens besonders geeignete Katalysatoren sind die Schalenkatalysatoren S1 (Stöchiometrie: $Mo_{12}V_3W_{1,2}Cu_{2,4}O_n$) und S7 (Stöchiometrie: $Mo_{12}V_3W_{1,2}Cu_{1,6}Ni_{0,8}O_n$) aus der DE-A 4442346 mit einem Aktivmassenanteil von 27 Gew.-% und einer Schalendicke von 230 pm, der Schalenkatalysator aus Herstellungsbeispiel 5 der DE-A 10046928 (Stöchiometrie: $Mo_{12}V_3W_{1,2}Cu_{2,4}O_n$) mit einem Aktivmassenanteil von 20 Gew.-%, die Schalenkatalysatoren gemäß den Beispielen 1 bis 5 aus der DE-A 19815281, jedoch ebenso wie die vorstehend genannten Schalenkatalysatoren für die zweite Reaktionsstufe auf Trägerringe der Geometrie 7 mm x 3 mm x 4 mm (Außendurchmesser x Länge x Innendurchmesser) mit einem Aktivmassenanteil von 20 Gew.-% (bezogen auf die Gesamtmasse des Schalenkatalysators) aufgebracht, sowie ein Schalenkatalysator mit zweiphasiger Aktivmasse der Stöchiometrie $(Mo_{10,4}V_3W_{1,2}O_x)$ $(CuMo_{0,5}W_{0,5}O_4)_{1,6}$, und hergestellt gemäß der DE-A 19736105 und einem Aktivmassenanteil von 20 Gew.-% auf den vorgenannten 7 mm x 3 mm x 4 mm Träger aufgebracht.

Die vorstehend für die erfindungsgemäße Reaktionsstufe empfohlenen Katalysatoren sind für die erfindungsgemäße Reaktionsstufe aber auch dann geeignet, wenn man alles beibehält und nur die Trägergeometrie auf 5 mm x 3 mm x 1,5 mm abändert (Au-ßendurchmesser x Länge x Innendurchmesser). Ferner können die genannten Multimetalloxide aber auch in Form der entsprechenden Vollkatalysatorringe in der erfindungsgemäßen Reaktionsstufe eingesetzt werden.

Erfindungsgemäß wesentlich ist, dass die Festbettkatalysatorschüttung aus wenigstens zwei räumlich aufeinander folgenden Festbettkatalysatorschüttungszonen besteht, wobei die volumenspezifische Aktivität innerhalb einer Festbettkatalysatorschüttungszone im wesentlichen konstant ist und in Strömungsrichtung des Reaktionsgasgemisches beim Übergang von einer Festbettkatalysatorschüttungszone in eine andere Festbettkatalysatorschüttungszone sprunghaft zunimmt.

Die volumenspezifische (d.h., die auf die Einheit des jeweiligen Schüttungsvolumens normierte) Aktivität einer Festbettkatalysatorschüttungszone kann nun in über die Festbettkatalysatorschüttungszone im wesentlichen konstanter Weises dadurch eingestellt werden, dass man von einer Grundmenge einheitlich hergestellter Katalysatorformkörper ausgeht (ihre Schüttung entspricht der maximal erzielbaren volumenspezifischen Aktivität) und diese in der jeweiligen Festbettkatalysatorschüttungszone mit sich bezüglich der heterogen katalysierten partiellen Gasphasenoxidation im wesentlichen inert verhaltenden Formkörpern (Verdünnungsformkörper) homogen verdünnt. Je höher der Anteil der Verdünnungsformkörper gewählt wird, desto geringer ist die in einem bestimmten Volumen der Schüttung enthaltene Aktivmasse bzw. Katalysatoraktivität. Als Materialien für solche inerten Verdünnungsformkörper kommen prinzipiell alle diejenigen in Betracht, die sich auch als Trägermaterial für erfindungsgemäß geeignete Schalenkatalysatoren eignen.

Als solche Materialien kommen z.B. poröse oder unporöse Aluminiumoxide, Siliciumdioxid, Thoriumdioxid, Zirkondioxid, Siliciumcarbid, Silikate wie Magnesium- oder Aluminiumsilikat oder der bereits erwähnte Steatit (z.B. Steatit C-220 der Fa. CeramTec) in Betracht.

Die Geometrie solcher inerter Verdünnungsformkörper kann im Prinzip beliebig sein. D.h., es können beispielsweise Kugeln, Polygone, Vollzylinder oder auch Ringe sein. Erfindungsgemäß bevorzugt wird man als inerte Verdünnungsformkörper solche wählen, deren Geometrie derjenigen der mit ihnen zu verdünnenden Katalysatorformkörper entspricht.

Erfindungsgemäß günstig ist es, wenn sich die chemische Zusammensetzung der verwendeten Aktivmasse über die gesamte Festbettkatalysatorschüttung nicht verändert. D.h., die für einen einzelnen Katalysatorformkörper verwendete Aktivmasse kann zwar ein Gemisch aus verschiedenen, die Elemente Mo und V enthaltenden, Multimetalloxiden sein, für alle Katalysatorformkörper der Festbettkatalysatorschüttung ist dann jedoch das gleiche Gemisch zu verwenden.

Eine in Strömungsrichtung des Reaktionsgasgemisches über die Festbettkatalysatorschüttung zonenweise zunehmende volumenspezifische Aktivität lässt sich für das erfindungsgemäße Verfahren somit in einfacher Weise z.B. dadurch einstellen, dass man die Schüttung in einer ersten Festbettkatalysatorschüttungszone mit einem hohen Anteil an inerten Verdünnungsformkörpern bezogen auf eine Sorte von Katalysatorformkörpern beginnt, und dann diesen Anteil an Verdünnungsformkörpern in Strömungsrichtung zonenweise verringert.

Eine zonenweise Zunahme der volumenspezifischen Aktivität ist aber auch z.B. dadurch möglich, dass man bei gleich bleibender Geometrie und Aktivmassenart eines Schalenkatalysatorformkörpers zonenweise die Dicke der auf dem Träger aufgebrachten Aktivmassenschicht erhöht oder in einem Gemisch aus Schalenkatalysatoren mit gleicher Geometrie aber mit unterschiedlichem Gewichtsanteil der Aktivmasse zonenweise den Anteil an Katalysatorformkörpern mit höherem Aktivmassengewichtsanteil steigert. Alternativ kann man auch die Aktivmassen selbst verdünnen, indem man bei der Aktivmassenherstellung z.B. in das zu calcinierende Trockengemisch aus Ausgangsverbindungen inerte verdünnend wirkende Materialien wie hochgebranntes Siliciumdioxid einarbeitet. Unterschiedliche Zusatzmengen an verdünnend wirkendem Material führen automatisch zu unterschiedlichen Aktivitäten. Je mehr verdünnend wirkendes Material zugesetzt wird, desto geringer wird die resultierende Aktivität sein. Analoge Wirkung lässt sich z.B. auch dadurch erzielen, dass man bei Mischungen aus Vollkatalysatoren und aus Schalenkatalysatoren (bei identischer Aktivmasse) in entsprechender Weise das Mischungsverhältnis verändert. Des weiteren lässt sich eine Variation der volumenspezifischen Aktivität durch den Einsatz von Katalysatorgeometrien mit unterschiedlicher Schüttdichte erzielen (z.B. bei Vollkatalysatoren mit identischer Aktivmassenzusammensetzung der verschiedenen Geometrien). Selbstredend lassen sich die beschriebenen Varianten auch kombiniert anwenden.

Natürlich können für die Festbettkatalysatorschüttung aber auch Mischungen aus Katalysatoren mit chemisch unterschiedlicher Aktivmassenzusammensetzung und als Folge dieser verschiedenen Zusammensetzung unterschiedlicher Aktivität verwendet werden. Diese Mischungen können wiederum zonenweise in ihrer Zusammensetzung variiert und/oder mit unterschiedlichen Mengen inerter Verdünnungsformkörper verdünnt werden.

Vorab und/oder im Anschluss an die Festbettkatalysatorschüttung können sich ausschließlich aus Inertmaterial (z.B. nur Verdünnungsformkörper) bestehende Schüttungen befinden (sie werden in dieser Schrift begrifflich nicht der Festbettkatalysatorschüttung zugerechnet, da sie keine Formkörper enthalten, die Multimetalloxidaktivmasse aufweisen). Dabei können die für die Inertschüttung verwendeten Verdünnungsformkörper die gleiche Geometrie wie die in der Festbettkatalysatorschüttung verwendeten Katalysatorformkörper aufweisen. Die Geometrie der für die Inertschüttung verwendeten Verdünnungsformkörper kann aber auch von der vorgenannten Geometrie der Katalysatorformkörper verschieden sein (z.B. kugelförmig anstatt ringförmig).

Häufig weisen die für solche Inertschüttungen verwendeten Formkörper die ringförmige Geometrie 7 mm x 7 mm x 4 mm (Außendurchmesser x Länge x Innendurchmesser) oder die kugelförmige Geometrie mit dem Durchmesser d = 4 - 5 mm auf. Die Temperaturzonen A und B können sich beim erfindungsgemäßen Verfahren auch auf die Inertschüttungen erstrecken. Erfindungsgemäß vorteilhaft erfassen sowohl die Temperaturzone A als auch die Temperaturzone B jeweils nicht mehr als drei Festbettkatalysatorschüttungszonen (erfindungsgemäß zwingend wird wenigstens eine Festbettkatalysatorschüttungszone von beiden Temperaturzonen erfasst).

Erfindungsgemäß besonders vorteilhaft umfasst die gesamte Festbettkatalysatorschüttung nicht mehr als fünf, zweckmäßig nicht mehr als vier bzw. drei Festbettkatalysator schüttungszonen.

Beim Übergang von einer Festbettkatalysatorschüttungszone in eine andere Festbettkatalysatorschüttungszone (in Strömungsrichtung des Reaktionsgasgemisches) sollte (bei einheitlicher Aktivmasse über die gesamte Festbettkatalysatorschüttung) die volumenspezifische Aktivmasse (d.h., das Gewicht der im Einheitsschüttungsvolumen enthaltenen Multimetalloxidmasse) erfindungsgemäß zweckmäßig um wenigstens 5 Gew.-%, bevorzugt um wenigstens 10 Gew.-% zunehmen (dies gilt insbesondere auch bei einheitlichen Katalysatorformkörpern über die gesamte Festbettkatalysatorschüttung). In der Regel wird diese Zunahme beim erfindungsgemäßen Verfahren nicht mehr als 50 Gew.-%, meist nicht mehr als 40 Gew.-% betragen. Ferner sollte bei einheitlicher Aktivmasse über die gesamte Festbettkatalysatorschüttung der Unterschied in der volumenspezifischen Aktivmasse derjenigen Festbettkatalysatorschüttungszone mit der geringsten volumenspezifischen Aktivität und derjenigen Festbettkatalysatorschüttungszone mit der höchsten volumenspezifischen Aktivität erfindungsgemäß vorteilhaft nicht mehr als 50 Gew.-%, vorzugsweise nicht mehr als 40 Gew.-% und besonders bevorzugt nicht mehr als 30 Gew.-% betragen.

Häufig wird beim erfindungsgemäßen Verfahren die Festbettkatalysatorschüttung aus nur zwei Festbettkatalysatorschüttungszonen bestehen.

Erfindungsgemäß bevorzugt ist die in Strömungsrichtung des Reaktionsgemisches letzte Festbettkatalysatorschüttungszone unverdünnt. D.h., sie besteht vorzugsweise ausschließlich aus Katalysatorformkörpern. Im Bedarfsfall kann sie auch aus einer Schüttung aus Katalysatorformkörpern bestehen, deren volumenspezifische Aktivität z.B. durch Verdünnung mit Inertmaterial abgesenkt, z.B. um 10 %, ist. Besteht die Festbettkatalysatorschüttung nur aus zwei Festbettkatalysatorschüttungszonen, ist es erfindungsgemäß in der Regel vorteilhaft (wie ganz allgemein beim erfindungsgemä-ßen Verfahren), wenn die Festbettkatalysatorschüttungszone mit der höchsten volumenspezifischen Aktivität bis in die Temperaturzone A hineinragt (insbesondere dann, wenn in der Temperaturzone A und in der Temperaturzone B die Temperierung mittels eines fließenden Wärmeträgers erfolgt, der jeweils im Gegenstrom zum Reaktionsgasgemisch strömt).

Besteht die Festbettkatalysatorschüttung nur aus drei Festbettkatalysatorschüttungszonen, ist es erfindungsgemäß in der Regel gleichfalls vorteilhaft, wenn die Festbettkatalysatorschüttungszone mit der höchsten volumenspezifischen Aktivität bis in die Temperaturzone A hineinragt (insbesondere dann, wenn in der Temperaturzone A und in der Temperaturzone B die Temperierung mittels eines fließenden Wärmeträgers erfolgt, der jeweils im Gegenstrom zum Reaktionsgasgemisch strömt).

Besteht die Festbettkatalysatorschüttung aus vier Festbettkatalysatorschüttungszonen, ist es erfindungsgemäß in der Regel vorteilhaft, wenn die Festbettkatalysatorschüttungszone mit der zweithöchsten volumenspezifischen Aktivität sowohl in die Temperaturzone A als auch in die Temperaturzone B hineinragt (insbesondere dann, wenn in der Temperaturzone A und in der Temperaturzone B die Temperierung mittels eines fließenden Wärmeträgers erfolgt, der jeweils im Gegenstrom zum Reaktionsgasgemisch strömt).

Im Fall einer Gleichstromführung von Reaktionsgasgemisch und Wärmeträgern in den Temperaturzonen A und B kann es erfindungsgemäß vorteilhaft sein, wenn die Festbettkatalysatorschüttungszone mit der höchsten volumenspezifischen Aktivität nicht in die Temperaturzone A hineinragt sondern erst hinter dem Übergang von der Temperaturzone A in die Temperaturzone B ihren Anfang hat.

Die volumenspezifische Aktivität zwischen zwei Festbettkatalysatorschüttungszonen lässt sich experimentell in einfacher Weise so differenzieren, dass unter identischen Randbedingungen (vorzugsweise den Bedingungen des ins Auge gefassten Verfahrens) über Festbettkatalysatorschüttungen der selben Länge, aber jeweils der Zusammensetzung der jeweiligen Festbettkatalysatorschüttungszone entsprechend, das gleiche Acrolein enthaltende Reaktionsgasausgangsgemisch geführt wird. Die höhere umgesetzte Menge an Acrolein weist die höhere volumenspezifische Aktivität aus.

Beträgt die Gesamtlänge der Festbettkatalysatorschüttung L, ist es erfindungsgemäß vorteilhaft, wenn sich im Bereich $X \pm L \cdot \dfrac{4}{100}$ bzw. im Bereich $X \pm L \cdot \dfrac{3}{100}$ bzw. im Bereich $X \pm L \cdot \dfrac{2}{100}$ kein Übergang von einer Festbettkatalysatorschüttungszone in eine andere Festbettkatalysatorschüttungszone befindet, wobei X der Ort innerhalb der Festbettkatalysatorschüttung ist, an dem der Übergang von der Temperaturzone A in die Temperaturzone B erfolgt.

Erfindungsgemäß bevorzugt ist beim erfindungsgemäßen Verfahren die Festbettkatalysatorschüttung in Strömungsrichtung des Reaktionsgasgemisches wie folgt strukturiert.

Zunächst auf einer Länge von 10 bis 60 %, bevorzugt 10 bis 50 %, besonders bevorzugt 20 bis 40 % und ganz besonders bevorzugt 25 bis 35 % (d.h., z.B. auf einer Länge von 0,70 bis 1,50 m, bevorzugt 0,90 bis 1,20 m), jeweils der Gesamtlänge der Festbettkatalysatorschüttung, ein homogenes Gemisch oder zwei (mit abnehmender Verdünnung) aufeinander folgende homogene Gemische aus Katalysatorformkörpern und Verdünnungsformkörpern (wobei beide vorzugsweise im wesentlichen die gleiche Geometrie aufweisen), wobei der Anteil der Verdünnungsformkörper so bemessen ist, dass die volumenspezifische Aktivmasse, bezogen auf eine nur aus den Katalysatorformkörpern bestehende Schüttung, um 10 bis 50 Gew.-%, vorzugsweise 20 bis 45 Gew.-% und besonders bevorzugt 25 bis 35 Gew.-% abgesenkt ist. Im Anschluß an diese erste bzw. an diese beiden ersten Zonen befindet sich dann erfindungsgemäß vorteilhaft bis zum Ende der Länge der Festbettkatalysatorschüttung (d.h., z.B. auf einer Länge von 2,00 bis 3,00 m, bevorzugt 2,50 bis 3,00 m) entweder eine nur in geringerem Umfang (als in der ersten bzw. in den ersten beiden Zonen) verdünnte Schüttung der Katalysatorformkörper, oder, ganz besonders bevorzugt, eine alleinige Schüttung derselben Katalysatorformkörper, die auch in der ersten Zone verwendet worden sind.

Das Vorgenannte trifft insbesondere dann zu, wenn in der Festbettkatalysatorschüttung als Katalysatorformkörper Schalenkatalysatorringe oder Schalenkatalysatorkugeln eingesetzt werden (insbesondere jene, die in dieser Schrift als bevorzugt aufgeführt werden). Mit Vorteil weisen im Rahmen der vorgenannten Strukturierung sowohl die Katalysatorformkörper bzw. deren Trägerringe als auch die Verdünnungsformkörper beim erfindungsgemäßen Verfahren im wesentlichen die Ringgeometrie 7 mm x 3 mm x 4 mm (Außendurchmesser x Länge x Innendurchmesser) auf.

Das oben Genannte trifft auch dann zu, wenn anstelle inerter Verdünnungsformkörper Schalenkatalysatorformkörper verwendet werden, deren Aktivmassenanteil um 2 bis 15 Gew.-% Punkte tiefer liegt als der Aktivmassenanteil der Schalenkatalysatorformkörper am Ende der Festbettkatalysatorschüttung.

Eine reine Inertmaterialschüttung, deren Länge, bezogen auf die Länge der Festbettkatalysatorschüttung, zweckmäßig 5 bis 20 % beträgt, leitet in Strömungsrichtung des Reaktionsgasgemisches in der Regel die Festbettkatalysatorschüttung ein. Sie wird normalerweise zum Temperieren des Reaktionsgasgemisches genutzt.

Erfindungsgemäß vorteilhaft erstreckt sich nun bei den vorgenannten Schüttungen die Temperaturzone A (die sich erfindungsgemäß vorteilhaft auch auf die Vorschüttung des Inertmaterials erstreckt) noch auf 5 bis 20 %, häufig auf 5 bis 15 % der Länge der in Strömungsrichtung des Reaktionsgasgemisches letzten (volumenspezifisch aktivsten) Festbettkatalysatorschüttungszone.

In anwendungstechnisch zweckmäßiger Weise erfolgt die Durchführung der Reaktionsstufe des erfindungsgemäßen Verfahrens in einem Zweizonenrohrbündelreaktor, wie er z.B. in den DE-A's 19910508, 19948523, 19910506 und 19948241 beschrieben ist. Eine bevorzugte Variante eines erfindungsgemäß einsetzbaren Zweizonenrohrbündelreaktors offenbart die DE-C 2830765. Aber auch die in der DE-C 2513405, der US-A 3147084, der DE-A 2201528, der EP-A 383224 und der DE-A 2903218 offenbarten Zweizonenrohrbündelreaktoren sind für eine Durchführung der Reaktionsstufe des erfindungsgemäßen Verfahrens geeignet.

D.h., in einfachster Weise befindet sich die erfindungsgemäß zu verwendende Festbettkatalysatorschüttung (eventuell mit vor- und/oder nach angeordneten Inertschüttungen) in den Metallrohren eines Rohrbündelreaktors und um die Metallrohre werden zwei voneinander im wesentlichen räumlich getrennte Temperiermedien, in der Regel Salzschmelzen, geführt. Der Rohrabschnitt, über den sich das jeweilige Salzbad erstreckt, repräsentiert erfindungsgemäß eine Temperaturzone.

D.h., in einfacher Weise umströmt z.B. ein Salzbad A diejenigen Abschnitte der Rohre (die Temperaturzone A), in welchem sich die oxidative Umsetzung des Acroleins (beim einfachen Durchgang) bis zum Erreichen eines Umsatzwertes im Bereich von 45 bis 85 mol-% (bevorzugt 50 bis 85 mol-%, besonders bevorzugt 60 bis 85 mol-%) vollzieht und ein Salzbad B umströmt den Abschnitt der Rohre (die Temperaturzone B), in welchem sich die oxidative Anschlussumsetzung des Acroleins (beim einfachen Durchgang) bis zum Erreichen eines Umsatzwertes von wenigstens 90 mol-% vollzieht (bei Bedarf können sich an die erfindungsgemäß anzuwendenden Temperaturzonen A, B weitere Temperaturzonen anschließen, die auf individuellen Temperaturen gehalten werden).

Anwendungstechnisch zweckmäßig umfasst die Reaktionsstufe des erfindungsgemäßen Verfahrens keine weiteren Temperaturzonen. D.h., das Salzbad B umströmt zweckmäßig den Abschnitt der Rohre, in welchem sich die oxidative Anschlussumsetzung des Acroleins (beim einfachen Durchgang) bis zu einem Umsatzwert von ≥92 mol-%, oder ≥94 mol% oder ≥96 mol-% oder ≥98 mol-% und häufig sogar ≥99 mol-% oder mehr vollzieht.

Üblicherweise liegt der Beginn der Temperaturzone B hinter dem Heißpunktmaximum der Temperaturzone A.

Die beiden Salzbäder A, B können erfindungsgemäß relativ zur Strömungsrichtung des durch die Reaktionsrohre strömenden Reaktionsgasgemisches im Gleichstrom oder im Gegenstrom durch den die Reaktionsrohre umgebenden Raum geführt werden. Selbstverständlich kann erfindungsgemäß auch in der Temperaturzone A eine Gleichströmung und in der Temperaturzone B eine Gegenströmung (oder umgekehrt) angewandt werden.

Selbstredend kann man in allen vorgenannten Fallkonstellationen innerhalb der jeweiligen Temperaturzone der, relativ zu den Reaktionsrohren, erfolgenden Parallelströmung der Salzschmelze noch eine Querströmung überlagern, so dass die einzelne Temperaturzone einem wie in der EP-A 700714 oder in der EP-A 700893 beschriebenen Rohrbündelreaktor entspricht und insgesamt im Längsschnitt durch das Kontaktrohrbündel ein mäanderförmiger Strömungsverlauf des Wärmeaustauschmittels resultiert.

Üblicherweise sind in den vorgenannten Zweizonen-Rohrbündelreaktoren für die erfindungsgemäße Reaktionsstufe die Kontaktrohre aus ferritischem Stahl gefertigt und weisen in typischer Weise eine Wanddicke von 1 bis 3 mm auf. Ihr Innendurchmesser beträgt in der Regel 20 bis 30 mm, häufig 21 bis 26 mm. Ihre Länge beträgt zweckmäßig 3 bis 4, bevorzugt 3,5 m. In jeder Temperaturzone belegt die Festbettkatalysatorschüttung wenigstens 60 %, bzw. wenigstens 75 %, oder wenigstens 90 % der Länge der Zone. Die gegebenenfalls verbleibende Restlänge wird gegebenenfalls von einer Inertschüttung belegt. Anwendungstechnisch zweckmäßig beläuft sich die im Rohrbündelbehälter untergebrachte Anzahl an Kontaktrohren auf wenigstens 5000, vorzugsweise auf wenigstens 10000. Häufig beträgt die Anzahl der im Reaktionsbehälter untergebrachten Kontaktrohre 15000 bis 30000. Rohrbündelreaktoren mit einer oberhalb von 40000 liegenden Anzahl an Kontaktrohren bilden eher die Ausnahme. Innerhalb des Behälters sind die Kontaktrohre im Normalfall homogen verteilt angeordnet (bevorzugt 6 äquidistante Nachbarrohre pro Kontaktrohr), wobei die Verteilung zweckmäßig so gewählt wird, dass der Abstand der zentrischen Innenachsen von zueinander nächstliegenden Kontaktrohren (die so genannte Kontaktrohrteilung) 35 bis 45 mm beträgt (vgl. EP-B 468290).

Als Wärmeaustauschmittel eignen sich insbesondere fluide Temperiermedien. Besonders günstig ist die Verwendung von Schmelzen von Salzen wie Kaliumnitrat, Kaliumnitrit, Natriumnitrit und/oder Natriumnitrat, oder von niedrig schmelzenden Metallen wie Natrium, Quecksilber sowie Legierungen verschiedener Metalle.

In der Regel wird bei allen vorstehend erwähnten Konstellationen der Stromführung in den Zweizonenrohrbündelreaktoren der erfindungsgemäßen Reaktionsstufe die Fließgeschwindigkeit innerhalb der beiden erforderlichen Wärmeaustauschmittelkreisläufe so gewählt, dass die Temperatur des Wärmeaustauschmittels von der Eintrittstelle in die Temperaturzone bis zur Austrittstelle aus der Reaktionszone um 0 bis 15°C ansteigt. D.h., das vorgenannte ΔT kann erfindungsgemäß 1 bis 10°C, oder 2 bis 8°C oder 3 bis 6°C betragen.

Die Eintrittstemperatur des Wärmeaustauschmittels in die Temperaturzone A liegt erfindungsgemäß normalerweise im Bereich von 230 bis 320°C, bevorzugt im Bereich von 250 bis 300°C und besonders bevorzugt im Bereich von 260 bis 280°C. Bei Acroleinbelastungen der Festbettkatalysatorschüttung von ≥70 Nl/l • h und < 150 Nl/l • h wird die Eintrittstemperatur des Wärmeaustauschmittels in die Reaktionszone B erfindungsgemäß normalerweise ebenfalls im Bereich von 230 bis 320°C bzw. 280°C, gleichzeitig aber normalerweise erfindungsgemäß zweckmäßig ≥0°C bis ≤20°C oder ≤10°C, bzw. ≥0°C und ≤5°C, oder häufig ≥0°C und ≤3°C unterhalb der Eintrittstemperatur des in die Reaktionszone A eintretenden Wärmeaustauschmittels liegen. Bei Acroleinbelastungen der Festbettkatalysatorschüttung von ≥150 Nl/l • h und (in der Regel) ≤300 Nl/l • h (bzw. 600 Nl/l • h) wird die Eintrittstemperatur des Wärmeaustauschmittels in die Temperaturzone B erfindungsgemäß ebenfalls im Bereich von 230 bis 320°C, gleichzeitig aber normalerweise erfindungsgemäß zweckmäßig > 0°C und ≤40°C, oder ≥5°C und ≤35°C bzw. ≤30°C, oder ≥10°C und ≤25°C bzw. ≤20°C, oder 15°C oberhalb der Eintrittstemperatur des in die Temperaturzone A eintretenden Wärmeaustauschmittels liegen.

Es sei an dieser Stelle auch noch einmal darauf hingewiesen, dass für eine Durchführung der erfindungsgemäßen Reaktionsstufe des erfindungsgemäßen Verfahrens insbesondere auch der in der DE-AS 2201528 beschriebene Zweizonenrohrbündelreaktortyp verwendet werden kann, der die Möglichkeit beinhaltet, vom heißeren Wärmeaustauschmittel der Reaktionszone B eine Teilmenge an die Reaktionszone A abzuführen, um gegebenenfalls ein Anwärmen eines zu kalten Reaktionsgasausgangsgemisches oder eines kalten Kreisgases zu bewirken. Ferner kann die Rohrbündelcharakteristik innerhalb einer individuellen Temperaturzone wie in der EP-A 382 098 beschrieben gestaltet werden.

Normalerweise wird beim erfindungsgemäßen Verfahren Acrolein eingesetzt, das durch katalytische Gasphasenoxidation von Propen erzeugt wurde. In der Regel werden die Acrolein enthaltenden Reaktionsgase dieser Propenoxidation ohne Zwischenreinigung eingesetzt, weshalb das erfindungsgemäße Reaktionsgasausgangsgemisch auch geringe Mengen von z.B. nicht umgesetztem Propen oder von Nebenprodukten der Propenoxidation enthalten kann. Normalerweise muss dabei dem Produktgasgemisch der Propenoxidation noch der für die Acroleinoxidation erforderliche Sauerstoff zugesetzt werden.

Mit Vorteil wird eine solche dem erfindungsgemäßen Verfahren vorausgehende gasphasenkatalytische Oxidation von Propen zu Acrolein in Analogie zum erfindungsgemäßen Verfahren durchgeführt.

D.h., zur Durchführung des erfindungsgemäßen Verfahrens können zwei Zweizonenrohrbündelreaktoren hintereinander geschaltet oder zu einem Vierzonenrohrbündelreaktor verschmolzen werden, wie es z.B. in der WO 01/36364 beschrieben ist, wobei im ersten Zweizonenteil die Propenoxidation und im zweiten Zweizonenteil die erfindungsgemäße Acroleinoxidation durchgeführt wird.

Normalerweise befindet sich im zweiten Fall zwischen den Festbettkatalysatorschüttungen für die beiden Reaktionsstufen eine Inertschüttung. Allerdings kann auf eine solche Zwischeninertschüttung auch verzichtet werden.

Natürlich kann die Propenoxidationsstufe aber auch ein separater oder mit der nachfolgenden Acroleinoxidationsstufe in entsprechender Weise verschmolzener Einzonenrohrbündelreaktor sein.

Die Länge der Reaktionsrohre entspricht im Verschmelzungsfall vielfach der Summe der Längen der nicht verschmolzenen Rohrbündelreaktoren.

Der Propenanteil im Reaktionsgasausgangsgemisch für die dem erfindungsgemäßen Verfahren vorausgehende Reaktionsstufe kann dabei z.B. bei Werten von 4 bis 15 Vol.-%, häufig bei 5 bis 12 Vol.-% bzw. 5 bis 8 Vol.-% liegen (jeweils bezogen auf das Gesamtvolumen).

Häufig wird man das dem erfindungsgemäßen Verfahren vorausgehende Verfahren bei einem Propen : Sauerstoff : indifferente Gase (einschließlich Wasserdampf) Volumenverhältnis im Reaktionsgasausgangsgemisch von 1 : (1,0 bis 3,0) : (5 bis 25), vorzugsweise 1 : (1,4 bis 2,3) : (10 bis 15) durchführen. Im Regelfall wird das indifferente (inerte) Gas zu wenigstens 20 % seines Volumens aus molekularem Stickstoff bestehen. Es kann aber auch zu ≥30 Vol.-%, oder zu ≥40 Vol.-%, oder zu ≥50 Vol.-%, oder zu ≥60 Vol.-%, oder zu ≥70 Vol.-%, oder zur ≥80 Vol.-%, oder zu ≥90 Vol.-%, oder zu ≥95 Vol.-% aus molekularem Stickstoff bestehen (generell sollen in dieser Schrift inerte Verdünnungsgase solche sein ,die sich beim einmaligen Durchgang durch die jeweilige Reaktionsstufe zu weniger als 5 %, bevorzugt zu weniger als 2 % umsetzen; das sind neben molarem Stickstoff z.B. Gase wie Propan, Ethan, Methan, Pentan, Butan, $CO_2$, CO, Wasserdampf und/oder Edelgase). Natürlich kann das inerte Verdünnungsgas beim dem erfindungsgemäßen Verfahren vorausgehenden Verfahren auch zu bis zu 50 mol-%, bzw. bis zu 75 mol-% und mehr aus Propan bestehen. Bestandteil des Verdünnungsgases kann auch Kreisgas sein, wie es nach der Abtrennung der Acrylsäure aus dem Produktgasgemisch des erfindungsgemäßen Verfahrens verbleibt.

Als Katalysatoren für eine solche gasphasenkatalytische Propenoxidation kommen insbesondere jene der EP - A 15565, der EP - A 575897, der DE - A 19746210 und der DE - A 19855913 in Betracht.

Das beim erfindungsgemäßen Verfahren mitzuverwendende Inertgas kann zu ≥20 Vol.-%, oder zu ≥30 Vol.-%, oder zu ≥40 Vol.%, oder zu ≥50 Vol.%, oder zu ≥60 Vol.-%, oder zu ≥70 Vol.-%, oder zu ≥80 Vol.-%, oder zu ≥90 Vol.-%, oder zu ≥95 Vol.-% aus molekularem Stickstoff bestehen.

In zweckmäßiger Weise wird das inerte Verdünnungsgas beim erfindungsgemäßen Verfahren zu 5 bis 20 Gew.-% aus $H_2O$ und zu 70 bis 90 Vol.-% aus $N_2$ bestehen.

Bei Acroleinbelastungen oberhalb von 250 Nl/l • h wird für das erfindungsgemäße Verfahren die Mitverwendung von inerten Verdünnungsgasen (inerte Verdünnungsgase sollen generell solche sein, die sich beim einmaligen Durchgang zu weniger als 5 %, bevorzugt zu weniger als 2 % umsetzen) wie Propan, Ethan, Methan, Butan, Pentan, $CO_2$ CO, Wasserdampf und/oder Edelgasen empfohlen. Selbstverständlich können diese Gase aber auch bereits bei geringeren Belastungen mitverwendet werden. Auch ist die Anwendung eines nur aus einem oder mehreren der vorgenannten Gase bestehenden Inertgas möglich.

Normalerweise wird beim erfindungsgemäßen Verfahren die Acroleinbelastung den Wert von 600 Nl/l • h nicht überschreiten.

Als Quelle für den im Rahmen des erfindungsgemäßen Verfahrens erforderlichen molekularen Sauerstoff kommt sowohl Luft als auch an molekularem Stickstoff entreicherte Luft (z.B. ≥90 Vol.-% $O_2$, ≤10 Vol.-% $N_2$) in Betracht.

Der Acroleinanteil im Reaktionsgasausgangsgemisch für das erfindungsgemäße Verfahren kann z.B. bei Werten von 3 bis 15 Vol.-%, häufig bei 4 bis 10 Vol.-% bzw. 5 bis 8 Vol.-% liegen (jeweils bezogen auf das Gesamtvolumen).

Häufig wird man das erfindungsgemäße Verfahren mit einem im Reaktionsgasausgangsgemisch vorliegenden Acrolein : Sauerstoff : Wasserdampf : Inertgas - Volumenverhältnis (NI) von 1 : (1 bis 3): (0 bis 20) : (3 bis 30), vorzugsweise von 1 : (1 bis 3) : (0,5 bis 10) : (7 bis 10) ausführen.

Selbstredend kann man das erfindungsgemäße Verfahren aber auch mit einem im Reaktionsgasausgangsgemisch vorliegenden Acrolein : Sauerstoff : Wasserdampf : Sonstige - Volumenverhältnis (NI) von 1 : (0,9 bis 1,3) : (2,5 bis 3,5) : (10 bis 12) ausführen.

An dieser Stelle sei noch festgehalten, dass als Aktivmassen für die Festbettkatalysatorschüttung des erfindungsgemäßen Verfahrens auch die Multimetalloxidmassen der DE-A 10261186 günstig sind.

Erfindungsgemäß günstige Ausgestaltungen eines Zweizonenrohrbündelreaktors für eine der erfindungsgemäßen Reaktionsstufe vorausgehende Propenpartialoxidationsstufe können wie folgt beschaffen sein (die konstruktive Detailgestaltung kann wie in den Gebrauchsmusteranmeldungen 202 19 277.6, 2002 19 278.4 und 202 19 279.2 bzw. in den PCT-Anmeldungen PCT/EP02/14187, PCT/EP02/14188 oder PCT/EP02/14189 erfolgen):

Kontaktrohre:

|  |  |
|---|---|
| Material der Kontaktrohre: | ferritischer Stahl; |
| Abmessungen der Kontaktrohre: | z.B. 3500 mm Länge; |
|  | z.B. 30 mm Außendurchmesser, |
|  | z.B. 2 mm Wandstärke; |

Anzahl der Kontaktrohre im Rohrbündel: z.B. 30000, oder 28000, oder 32000, oder 34000; zusätzlich bis zu 10 Thermorohre (wie in EP-A 873 783 und EP-A 12 70 065 beschrieben), die wie die Kontaktrohre beschickt sind (schneckenartig von ganz außen nach innen drehend) z.B. der selben Länge und Wandstärke, aber mit einem Außendurchmesser von z.B. 33,4 mm und einer zentrierten Thermohülse von z.B. 8 mm Außendurchmesser und z.B. 1 mm Wandstärke;

Reaktor (Material wie die Kontaktrohre):

Zylinderförmiger Behälter eines Innendurchmessers von 6000 - 8000 mm;

Reaktorhauben plattiert mit Edelstahl des Typs 1,4541; Plattierungsdicke: einige mm;

ringförmig angeordnetes Rohrbündel, z.B. mit einem freien zentralen Raum;

Durchmesser des zentralen freien Raumes: z.B. 1000 - 2500 mm (z.B. 1200 mm, oder 1400 mm, oder 1600 mm, oder 1800 mm, oder 2000 mm, oder 2200 mm, oder 2400 mm);

normalerweise homogene Kontaktrohrverteilung im Rohrbündel (6 äquidistante Nachbarrohre pro Kontaktrohr), Anordnung in gleichseitigem Dreieck, Kontaktrohrteilung (Abstand der zentrischen Innenachsen von zueinander nächstliegenden Kontaktrohren): 35 - 45 mm, z.B. 36 mm, oder 38 mm, oder 40 mm, oder 42 mm, oder 44 mm;

die Kontaktrohre sind mit ihren Enden in Kontaktrohrböden (oberer Boden und unterer Boden z.B. mit einer Dicke von 100 - 200 mm) abdichtend befestigt und münden am oberen Ende in eine mit dem Behälter verbundene Haube, die einen Zulass für das Reaktionsgasausgangsgemisch aufweist; ein z.B. auf der halben Kontaktrohrlänge befindliches Trennblech einer Dicke von 20 - 100 mm, teilt den Reaktorraum symmetrisch in zwei Temperaturzonen A' (obere Zone) und B' (untere Zone); jede Temperaturzone wird durch eine Umlenkscheibe in 2 äquidistante Längsabschnitte geteilt;

die Umlenkscheibe weist bevorzugt Ringgeometrie auf; die Kontaktrohre sind mit Vorteil am Trennblech abdichtend befestigt; an den Umlenkscheiben sind sie nicht abdichtend befestigt, so dass die Querströmungsgeschwindigkeit der Salzschmelze innerhalb einer Zone möglichst konstant ist;

jede Zone wird durch eine eigene Salzpumpe mit Salzschmelze als Wärmeträger versorgt; die Zufuhr der Salzschmelze ist z.B. unterhalb der Umlenkscheibe und die Entnahme ist z.B. oberhalb der Umlenkscheibe;

aus beiden Salzschmelzekreisen wird z.B. ein Teilstrom entnommen und z.B. in einem gemeinsamen oder zwei getrennten indirekten Wärmetauschern abgekühlt (Dampferzeugung);

im ersten Fall wird der abgekühlte Salzschmelzestrom aufgeteilt, mit dem jeweiligen Reststrom vereinigt und durch die jeweilige Pumpe in den entsprechenden Ringkanal, der die Salzschmelze über den Behälterumfang verteilt, in den Reaktor gedrückt;

durch im Reaktormantel befindliche Fenster gelangt die Salzschmelze zum Rohrbündel; die Einströmung erfolgt z.B. in radialer Richtung zum Rohrbündel;

die Salzschmelze fließt in jeder Zone der Vorgabe des Umlenkblechs folgend z.B. in der Abfolge

- von außen nach innen,
- von innen nach außen,

um die Kontaktrohre;

durch um den Behälterumfang angebrachte Fenster sammelt sich die Salzschmelze an jedem Zonenende in einem um den Reaktormantel angebrachten Ringkanal, um einschließlich Teilstromkühlung im Kreis gepumpt zu werden;

über jede Temperaturzone wird die Salzschmelze von unten nach oben geführt.

Das Reaktionsgasgemisch verlässt den Reaktor der der erfindungsgemäßen Reaktionsstufe voraus geschalteten Reaktionsstufe mit einer Temperatur wenige Grad höher als die Salzbadeintrittstemperatur dieses Reaktors. Das Reaktionsgasgemisch wird für die Weiterverarbeitung zweckmäßigerweise in einem separaten Nachkühler, der diesem Reaktor nachgeschaltet ist, auf 220˚C bis 280˚C, bevorzugt 240˚C bis 260˚C abgekühlt.

Der Nachkühler ist in der Regel unterhalb des unteren Rohrbodens angeflanscht und besteht normalerweise aus Rohren mit ferritischem Stahl. In die Rohre des Nachkühlers sind mit Vorteil innen Edelstahl-Blechspiralen, die teil- oder vollgewendelt sein können, zur Verbesserung des Wärmeübergangs eingeführt.

Salzschmelze:

Als Salzschmelze kann ein Gemisch aus 53 Gew.-% Kaliumnitrat, 40 Gew.-% Natriumnitrit und 7 Gew.-% Natriumnitrat verwendet werden; beide Reaktionszonen und der Nachkühler wenden mit Vorteil eine Salzschmelze derselben Zusammensetzung an; die in den Reaktionszonen umgepumpte Salzmenge kann je Zone ca. 10000 $m^3$/h betragen.

Stromführung:

das Reaktionsgasausgangsgemisch für die Propenoxidation strömt zweckmäßig von oben nach unten durch den Reaktor, während die unterschiedlich temperierten Salzschmelzen der einzelnen Zonen zweckmäßig von unten nach oben gefördert werden;

Kontaktrohr- und Thermorohrbeschickung (von oben nach unten) z.B.:

Abschnitt 1:    50 cm Länge
Steatitringe der Geometrie 7 mm x 7 mm x 4 mm (Außendurchmesser x Länge x Innendurchmesser) als Vorschüttung.

Abschnitt 2:    140 cm Länge
Katalysatorbeschickung mit einem homogenen Gemisch aus 30 Gew.-% an Steatitringen der Geometrie 5 mm x 3 mm x 2 mm (Außendurchmesser x Länge x Innendurchmesser) und 70 Gew.-% Vollkatalysator aus Abschnitt 3.

Abschnitt 3:    160 cm Länge
Katalysatorbeschickung mit ringförmigem (5 mm x 3 mm x 2 mm = Außendurchmesser x Länge x Innendurchmesser) Vollkatalysator gemäß Beispiel 1 der DE-A 10046957 (Stöchiometrie: $[Bi_2W_2O_9$ x 2 $WO_3]_{0,5}$ $[Mo_{12}CO_{5,5}Fe_{2,94}Si_{1,59}K_{0,08}O_x]_1$).

Erfindungsgemäß günstige Ausgestaltungen eines Zweizonenrohrbündelreaktors für die erfindungsgemäße Acroleinreaktionsstufe können wie folgt beschaffen sein:

Alles wie beim Zweizonenrohrbündelreaktor für die Propenreaktionsstufe. Die Dicke des oberen und unteren Kontaktrohrbodens beträgt jedoch häufig 100 - 200 mm, z.B. 110 mm, oder 130 mm, oder 150 mm, oder 170 mm, oder 190 mm.

Der Nachkühler entfällt; statt dessen münden die Kontaktrohre mit ihren unteren Öffnungen in eine am unteren Ende mit dem Behälter verbundene Haube mit Auslass für das Produktgasgemisch; die obere Temperaturzone ist die Zone A und die untere Temperaturzone ist die Temperaturzone B. Zwischen Auslass "Nachkühler" und Einlass "Reaktor für die erfindungsgemäße Reaktionsstufe" besteht zweckmäßig eine Zufuhrmöglichkeit für komprimierte Luft.

Die Kontaktrohr- und Thermorohrbeschickung (von oben nach unten) kann z.B. wie folgt sein:

Abschnitt 1:   20 cm Länge
Steatitringe der Geometrie 7 mm x 7 mm x 4 mm (Außendurchmesser x Länge x Innendurchmesser) als Vorschüttung.

Abschnitt 2:   90 cm Länge
Katalysatorbeschickung mit einem homogenen Gemisch aus 30 Gew.-% an Steatit-Ringen der Geometrie 7 mm x 3 mm x 4 mm (Außendurchmesser x Länge x Innendurchmesser) und 70 Gew.-% Schalenkatalysator aus Abschnitt 4.

Abschnitt 3:   50 cm Länge
Katalysatorbeschickung mit einem homogenen Gemisch aus 20 Gew.-% an Steatit-Ringen der Geometrie 7 mm x 3 mm x 4 mm (Außendurchmesser x Länge x Innendurchmesser) und 80 Gew.-% Schalenkatalysator aus Abschnitt 4.

Abschnitt 4:   190 cm Länge
Katalysatorbeschickung mit ringförmigem (7 mm x 3 mm x 4 mm = Außendurchmesser x Länge x Innendurchmesser) Schalenkatalysator gemäß Herstellungsbeispiel 5 der DE-A 10046928 (Stöchiometrie: $Mo_{12}V_3W_{1,2}Cu_{2,4}O_x$).

Alternativ kann die Propenstufen-Kontaktrohr und Thermorohrbeschickung (von oben nach unten) auch so aussehen:

Abschnitt 1:   50 cm Länge
Steatitringe der Geometrie 7 mm x 7 mm x 4 mm (Außendurchmesser x Länge x Innendurchmesser) als Vorschüttung.

Abschnitt 2:   300 cm Länge
Katalysatorbeschickung mit ringförmigem (5 mm x 3 mm x 2 mm = Außendurchmesser x Länge x Innendurchmesser) Vollkatalysator gemäß Beispiel 1 der DE-A 10046957 (Stöchiometrie: $[Bi_2W_2O_9$ x $2WO_3]_{0,5}$ • $[Mo_{12}Co_{5,6}Fe_{2,94}Si_{1,59}K_{0,08}O_x]_1$).

Die Acroleinstufen-Kontaktrohr- und Thermorohrbeschickung kann (von oben nach unten) auch so aussehen:

Abschnitt 1:   20 cm Länge
Steatitringe der Geometrie 7 mm x 7 mm x 4 mm (Außendurchmesser x Länge x Innendurchmesser) als Vorschüttung.

Abschnitt 2:   140 cm Länge
Katalysatorbeschickung mit einem homogenen Gemisch aus 25 Gew.-% an Steatit-Ringen der Geometrie 7 mm x 3 mm x 4 mm (Außendurchmesser x Länge x Innendurchmesser) und 75 Gew.-% Schalenkatalysator aus Abschnitt 3.

Abschnitt 3:   190 cm Länge
Katalysatorbeschickung mit ringförmigem (7 mm x 3 mm x 4 mm = Außendurchmesser x Länge x Innendurchmesser) Schalenkatalysator gemäß Herstellungsbeispiel 5 der DE-A 10046928 (Stöchiometrie: $Mo_{12}V_3W_{1,2}Cu_{2,4}O_x$).

In allen genannten Propenstufenbeschickungen kann der Vollkatalysator aus Beispiel 1 der DE-A 10046957 auch ersetzt werden durch:

a) einen Katalysator gemäß Beispiel 1 c aus der EP-A 15565 oder einen gemäß diesem Beispiel herzustellenden Katalysator, der jedoch die Aktivmasse $Mo_{12}Ni_{6,5}Zn_2Fe_2Bi_1P_{0,0065}K_{0,06}O_x$ • 10 $SiO_2$ aufweist;

b) Beispiel Nr. 3 aus der DE-A 19855913 als Hohlzylindervollkatalysator der Geometrie 5 mm x 3 mm x 2 mm bzw. 5 mm x 2 mm x 2 mm;

c) Multimetalloxid II- Vollkatalysator gemäß Beispie 1 der DE-A 19746210;

d) einen der Schalenkatalysatoren 1, 2 und 3 aus der DE-A 10063162, jedoch bei gleicher Schalendicke auf Trägerringe der Geometrie 5 mm x 3 mm x 1,5 mm bzw. 7 mm x 3 mm x 1,5 mm aufgebracht;

e) einen Schalenkatalysator gemäß den Ausführungsbeispielen der DE-A 19815281 jedoch bei gleicher Schalendicke auf Trägerringe der Geometrie 7 mm x 3 mm x 4 mm, oder 8 mm x 6 mm x 4 mm (stets Außendurchmesser x Länge x Innendurchmesser) aufgebracht.

In vorgenannter Auflistung wird als Trägermaterial bevorzugt Steatit der Fa. CeramTec eingesetzt (Typ: C220).

In allen vorgenannten erfindungsgemäßen Acroleinstufenbeschickungen kann der Schalenkatalysator gemäß Herstellungsbeispiel 5 der DE-A 10046928 ersetzt werden durch:

a) Schalenkatalysator S1 oder S7 aus der DE-A 4442346 mit einem Aktivmassenanteil von 27 Gew.-% und einer Schalendicke von 230 $\mu$m;

b) einem Schalenkatalysator gemäß den Beispielen 1 bis 5 aus der DE-A 19815281, jedoch auf Trägerringe der Geometrie 7 mm x 3 mm x 4 mm mit einem Aktivmassenanteil von 20 Gew.-% aufgebracht;

c) Schalenkatalysator mit zweiphasiger Aktivmasse der Stöchiometrie $(Mo_{10,4}V_3W_{1,2}O_x)$ $(CuMo_{0,5}W_{0,5}O_4)_{1,6}$, hergestellt gemäß DE-A 19736105 und mit einem Aktivmassenanteil von 20 Gew.-% auf den vorgenannten 7 mm x 3 mm x 4 mm Träger aufgebracht.

Im Übrigen werden die Festbettkatalysatorschüttung für die Propenoxidationsstufe und die Festbettkatalysatorschüttung für die erfindungsgemäße Acroleinoxidationsstufe zweckmäßig so gewählt (z.B. durch Verdünnung mit z.B. Inertmaterial), dass der Temperaturunterschied zwischen dem Heißpunktmaximum des Reaktionsgasgemisches in den einzelnen Reaktionszonen und der jeweiligen Temperatur der Reaktionszone in der Regel 80˚C nicht überschreitet. Meist beträgt dieser Temperaturunterschied ≤ 70˚C, häufig liegt er bei 20 bis 70˚C, vorzugsweise ist dieser Temperaturunterschied gering. Außerdem werden diese Festbettkatalysatorschüttungen aus Sicherheitsgründen in dem Fachmann an sich bekannter Weise so gewählt (z.B. durch Verdünnung mit z.B. Inertmaterial), dass die "peak-to-salt-temperature sensitivity" gemäß Definition in der EP-A 1106598 ≤9˚C, oder ≤7˚C, oder ≤5˚C, oder ≤3˚C beträgt.

Nachkühler und Reaktor für die Acroleinreaktionsstufe sind durch ein Verbindungsrohr verbunden, dessen Länge weniger als 25 m beträgt.

In den nachfolgenden Beispielen sowie in der vorstehenden Reaktoranordnung können in der Acroleinreaktionsstufe die ringförmigen Verdünnungsformkörper und die ringförmigen Katalysatorformkörper auch durch kugelförmige Verdünnungsformkörper und kugelförmige Katalysatorformkörper (jeweils mit Radius 2 bis 5 mm und mit einem Aktivmassenanteil von 10 bis 30 Gew.-%, häufig 10 bis 20 Gew.-%) ersetzt werden.

Beispiele

Ein Reaktionsrohr (V2A Stahl; 30 mm Außendurchmesser, 2 mm Wandstärke, 26 mm Innendurchmesser, Länge: 350 cm, sowie ein in der Reaktionsrohrmitte zentriertes Thermorohr (4 mm Außendurchmesser) zur Aufnahme eines Thermoelements mit dem die Temperatur im Reaktionsrohr auf seiner gesamten Länge ermittelt werden kann) wird von oben nach unten wie folgt beschickt:

Abschnitt 1: 20 cm Länge
Steatitringe der Geometrie 7 mm x 7 mm x 4 mm (Außendurchmesser x Länge x Innendurchmesser) als Vorschüttung.

Abschnitt 2: 90 cm Länge
Katalysatorbeschickung mit einem homogenen Gemisch aus 30 Gew.-% an Steatit-Ringen der Geometrie 7 mm x 3 mm x 4 mm (Außendurchmesser x Länge x Innendurchmesser) und 70 Gew.-% Schalenkatalysator aus Abschitt 4.

Abschnitt 3: 50 cm Länge
Katalysatorbeschickung mit einem homogenen Gemisch aus 20 Gew.-% an Steatit-Ringen der Geometrie 7 mm x 3 mm x 4 mm (Außendurchmesser x Länge x Innendurchmesser) und 80 Gew.-% Schalenkatalysator aus Abschnitt 4.

Abschnitt 4: 190 cm Länge

Katalysatorbeschickung mit ringförmigem (7 mm x 3 mm x 4 mm = Außendurchmesser x Länge x Innendurchmesser) Schalenkatalysator gemäß Herstellungsbeispiel 5 der DE-A 10046928 (Stöchiometrie: $Mo_{12}V_3W_{1,2}Cu_{2,40}O_x$).

Von oben nach untern werden die ersten 175 cm mittels eines im Gegenstrom gepumpten Salzbades A thermostatisiert. Die zweiten 175 cm werden mittels eines im Gegenstrom gepumpten Salzbades B thermostatisiert.

Gasphasenoxidation:

Das vorstehend beschriebene Reaktionsrohr wird mit einem Reaktionsgasausgangsgemisch der nachfolgenden Zusammensetzung kontinuierlich beschickt, wobei die Belastung und die Thermostatisierung des Reaktionsrohres variiert werden:

5,5 Vol.-% Acrolein,
0,3 Vol.-% Propen,
6,0 Vol.-% molekularer Sauerstoff,
0,4 Vol.-% CO,
0,8 Vol.% $CO_2$,
9,0 Vol.% Wasser und,
78,0 Vol.-% Stickstoff.

Das Reaktionsgasgemisch durchströmt das Reaktionsrohr von oben nach unten.

Der Druck am Eingang des Reaktionsrohres variiert in Abhängigkeit von der Acroleinbelastung zwischen 1,6 und 2,1 bar.

Dem Produktgasgemisch wird am Reaktionsrohrausgang eine kleine Probe für die gaschromatographische Analyse entnommen. Am Ende der Reaktionszone A befindet sich ebenfalls eine Analysenstelle.

Die Ergebnisse in Abhängigkeit von Belastungen und Salzbadtemperaturen zeigt die nachfolgende Tabelle 1 (der Buchstabe B in Klammern bedeutet Beispiel).

$T_A, T_B$, stehen für die Temperaturen der umgepumpten Salzbäder in den Reaktionszonen A und B.

$U_A^{AC}$ ist der Acroleinumsatz am Ende der Reaktionszone A in mol-%.

$\dot{U}_B^{AC}$ ist der Acroleinumsatz am Ende der Reaktionszone B in mol-%.

$S^{AA}$ ist die Selektivität der Acrylsäurebildung im Produktgasgemisch bezogen auf umgesetztes Acrolein in mol-%.

$T^{maxA}$, $T^{maxB}$ stehen für die höchste Temperatur des Reaktionsgasgemisches innerhalb der Reaktionszonen A und B in ˚C.

Tabelle 1

| Acroleinbelastung (Nl/l•h) | $T_A$ | $T_B$ | $T^{maxA}$ | $T^{maxB}$ | $U_A^{AC}$ | $U_B^{AC}$ | $S^{AA}$ |
|---|---|---|---|---|---|---|---|
| 106 (B) | 260 | 260 | 302 | 276 | 80,7 | 99,3 | 95,4 |
| 108 (B) | 262 | 259 | 312 | 275 | 84,8 | 99,3 | 95,8 |
| 152 (B) | 263 | 269 | 303 | 287 | 78,8 | 99,3 | 95,8 |

Vergleichsbeispiele

Alles wird wie in den Beispielen durchgeführt. Die Länge der Beschickungsabschnitte der Festbettkatalysatorbeschickung wird jedoch wie folgt geändert:

Abschnitt 3:    65 cm anstelle 50 cm.
Abschnitt 4:    175 cm anstelle 190 cm.

Die Ergebnisse zeigt die Tabelle 2. (V) bedeutet Vergleichsbeispiel.

Tabelle 2

| Acroleinbelastung (Nl/l•h) | $T_A$ | $T_B$ | $T^{maxA}$ | $T^{maxB}$ | $U_A^{AC}$ | $U_B^{AC}$ | $S^{AA}$ |
|---|---|---|---|---|---|---|---|
| 103 (V) | 265 | 260 | 323 | 276 | 80,8 | 99,3 | 95,2 |
| 106 (V) | 268 | 259 | 332 | 275 | 84,9 | 99,2 | 95,0 |
| 150 (V) | 267 | 269 | 326 | 287 | 78,6 | 99,3 | 95,3 |

Im Vergleich mit den Ergebnissen in Tabelle 1 werden bei vergleichbaren $U_B^{AC}$ Werten geringere $S^{AA}$ erzielt.

**Patentansprüche**

1. Verfahren der heterogen katalysierten partiellen Gasphasenoxidation von Acrolein zu Acrylsäure, bei dem man ein Acrolein, molekularen Sauerstoff und wenigstens ein Intertgas enthaltendes Reaktionsgasausgangsgemisch, das den molekularen Sauerstoff und das Acrolein in einem molekularen Verhältnis $O_2 : C_3H_4O \geq 0,5$ enthält, in einer Reaktionsstufe mit der Maßgabe über eine Festbettkatalysatorschüttung, deren Aktivmasse wenigstens ein die Elemente Mo und V enthaltendes Multimetalloxid ist, führt, dass

   - die Festbettkatalysatorschüttung in zwei räumlich aufeinanderfolgenden Temperaturzonen A, B angeordnet ist,
   - sowohl die Temperatur der Temperaturzone A als auch die Temperatur der Temperaturzone B eine Temperatur im Bereich von 230 bis 320˚C ist,
   - die Festbettkatalysatorschüttung aus wenigstens zwei räumlich aufeinanderfolgenden Festbettkatalysatorschüttungszonen besteht, wobei die volumenspezifische Aktivität innerhalb einer Festbettkatalysatorschüttungszone im wesentlichen konstant ist und in Strömungsrichtung des Reaktionsgasgemisches beim Übergang von einer Festbettkatalysatorschüttungszone in eine andere Festbettkatalysatorschüttungszone sprunghaft zunimmt,
   - sich die Temperaturzone A bis zu einem Umsatz des Acroleins von 45 bis 85 mol-% erstreckt,
   - bei einmaligem Durchgang des Reaktionsgasausgangsgemisches durch die gesamte Festbettkatalysatorschüttung der Acroleinumsatz $\geq$ 90 mol-% und die Selektivität der Acrylsäurebildung, bezogen auf umgesetztes Acrolein, $\geq$90 mol-% beträgt,
   - die zeitliche Abfolge, in der das Reaktionsgasgemisch die Temperaturzonen A, B durchströmt, der alphabetischen Abfolge der Temperaturzonen entspricht,
   - die Belastung der Festbettkatalysatorschüttung mit dem im Reaktionsgasausgangsgemisch enthaltenen Acrolein $\geq$ 70 Nl Acrolein/l Festbettkatalysatorschüttung • h beträgt, und
   - die Differenz $T^{maxA} - T^{maxB}$, gebildet aus der höchsten Temperatur $T^{maxA}$, die das Reaktionsgasgemisch innerhalb der Temperaturzone A aufweist, und der höchsten Temperatur $T^{maxB}$, die das Reaktionsgasgemisch innerhalb der Temperaturzone B aufweist, $\geq$0˚C beträgt,

   **dadurch gekennzeichnet, dass** der Übergang von der Temperaturzone A in die Temperaturzone B in der Festbettkatalysatorschüttung nicht mit einem Übergang von einer Festbettkatalysatorschüttungszone in eine andere Festbettkatalysatorschüttungszone zusammenfällt.

2. Verfahren nach Anspruch 1, **dadurch gekennzeichnet, dass** $T^{maxA} - T^{maxB} \geq$3˚C und $\leq$60˚C beträgt.

3. Verfahren nach Anspruch 1, **dadurch gekennzeichnet, dass** $T^{maxA} - T^{maxB} \geq$5˚C und $\leq$40˚C beträgt.

4. Verfahren nach einem der Ansprüche 1 bis 3, **dadurch gekennzeichnet, dass** die Acroleinbelastung der Festbettkatalysatorschüttung $\geq$70 Nl/l • h und < 150 Nl/l • h beträgt

5. Verfahren nach einem der Ansprüche 1 bis 3, **dadurch gekennzeichnet, dass** die Acroleinbelastung der Festbettkatalysatorschüttung $\geq$150 Nl/l • h und $\leq$300 Nl/l • h beträgt.

6. Verfahren nach einem der Ansprüche 1 bis 5, **dadurch gekennzeichnet, dass** die Temperatur der Reaktionszone A 250 bis 300˚C beträgt.

**7.** Verfahren nach einem der Ansprüche 1 bis 5, **dadurch gekennzeichnet, dass** die Temperatur der Reaktionszone A 260 bis 280˚C beträgt.

**8.** Verfahren nach einem der Ansprüche 1 bis 7, **dadurch gekennzeichnet, dass** sich die Temperaturzone A bis zu einem Umsatz des Acroleins von 50 bis 85 mol-% erstreckt.

**9.** Verfahren nach einem der Ansprüche 1 bis 8, **dadurch gekennzeichnet, dass** sich die Temperaturzone A bis zu einem Umsatz des Acroleins von 60 bis 85 mol-% erstreckt.

**10.** Verfahren nach einem der Ansprüche 1 bis 9, **dadurch gekennzeichnet, dass** das Verhältnis $O_2$: Acrolein im Reaktionsgasausgangsgemisch $\geq 1$ beträgt.

**11.** Verfahren nach einem der Ansprüche 1 bis 10, **dadurch gekennzeichnet, dass** die chemische Zusammensetzung der verwendeten Aktivmasse über die gesamt Festbettkatalysatorschüttung unverändert ist.

**12.** Verfahren nach einem der Ansprüche 1 bis 11, **dadurch gekennzeichnet, dass** die gesamte Festbettkatalysatorschüttung nicht mehr als vier Festbettkatalysatorschüttungszonen umfasst.

**13.** Verfahren nach einem der Ansprüche 1 bis 12, **dadurch gekennzeichnet, dass** bei einheitlicher Aktivmasse über die gesamte Festbettkatalysatorschüttung beim Übergang von einer Festbettkatalysatorschüttungszone in eine andere Festbettkatalysatorschüttungszone in Strömungsrichtung des Reaktionsgasgemisches die volumenspezifische Aktivmasse um wenigstens 5 Gew.-% zunimmt.

**14.** Verfahren nach einem der Ansprüche 1 bis 12, **dadurch gekennzeichnet, dass** bei einheitlicher Aktivmasse über die gesamte Festbettkatalysatorschüttung beim Übergang von einer Festbettkatalysatorschüttungszone in eine andere Festbettkatalysatorschüttungszone in Strömungsrichtung des Reaktionsgasgemisches die volumenspezifische Aktivmasse um wenigstens 10 Gew.-% zunimmt.

**15.** Verfahren nach einem der Ansprüche 1 bis 14, **dadurch gekennzeichnet, dass** bei einheitlicher Aktivmasse über die gesamte Festbettkatalysatorschüttung der Unterschied in der volumenspezfischen Aktivmasse derjenigen Festbettkatalysatorschüttungszone mit der geringsten volumenspezifischen Aktivität und derjenigen Festbettkatalysatorschüttungszone mit der höchsten volumenspezifischen Aktivität nicht mehr als 40 Gew.-% beträgt.

**16.** Verfahren nach einem der Ansprüche 1 bis 15, **dadurch gekennzeichnet, dass** die in Strömungsrichtung des Reaktionsgasgemisches letzte Festbettkatalysatorschüttungszone unverdünnt ist und nur aus Katalysatorformkörpern besteht.

**17.** Verfahren nach einem der Ansprüche 1 bis 16, **dadurch gekennzeichnet, dass** die Festbettkatalysatorschüttungszone mit der höchsten volumenspezifischen Aktivität bis in die Temperaturzone A hineinragt.

**18.** Verfahren nach einem der Ansprüche 1 bis 16, **dadurch gekennzeichnet, dass** die Festbettkatalysatorschüttung aus vier Festbettkatalysatorschüttungszonen besteht und die Festbettkatalysatorschüttungszone mit der zweithöchsten volumenspezifischen Aktivität sowohl in die Temperaturzone A als auch in die Temperaturzone B hineinragt.

**19.** Verfahren nach einem der Ansprüche 1 bis 18, **dadurch gekennzeichnet, dass** sich in der Festbettkatalysatorschüttung im Bereich $X \pm L \cdot \dfrac{4}{100}$ kein Übergang von einer Festbettkatalysatorschüttungszone in eine andere Festbettkatalysatorschüttung befindet, wobei L die Länge der Festbettkatalysatorschüttung und X die Stelle innerhalb der Festbettkatalysatorschüttung ist, wo der Übergang von der Temperaturzone A in die Temperaturzone B erfolgt.

**Claims**

**1.** A process for partially oxidizing acrolein to acrylic acid in the gas phase under heterogeneous catalysis by conducting a starting reaction gas mixture comprising acrolein, molecular oxygen and at least one inert gas, and comprises the molecular oxygen and the acrolein in a molar $O_2:C_3H_4O$ ratio of $\geq 0.5$ in a reaction stage over a fixed catalyst bed whose active composition is at least one multimetal oxide comprising the elements Mo and V, with the proviso that

- the fixed catalyst bed is arranged in two spatially successive temperature zones A, B,
- both the temperature of temperature zone A and the temperature of temperature zone B is a temperature in the range from 230 to 320°C,
- the fixed catalyst bed consists of at least two spatially successive fixed catalyst bed zones, and the volume-specific activity within one fixed catalyst bed zone is substantially constant and increases sharply in the flow direction of the reaction gas mixture at the transition from one fixed catalyst bed zone to another fixed catalyst bed zone,
- the temperature zone A extends up to a conversion of the acrolein of from 45 to 85 mol%,
- on single pass of the starting reaction gas mixture through the entire fixed catalyst bed, the acrolein conversion is $\geq 90$ mol% and the selectivity of acrylic acid formation based on converted acrolein is $\geq 90$ mol%,
- the sequence in time in which the reaction gas mixture flows through the temperature zones A, B corresponds to the alphabetic sequence of the temperature zones,
- the hourly space velocity of the acrolein present in the starting reaction gas mixture on the fixed catalyst bed is $\geq 70$ 1 (STP) of acrolein/l of fixed bed catalyst h, and
- the difference $T^{maxA} - T^{maxB}$, formed from the highest temperature $T^{maxA}$ which the reaction gas mixture has within temperature zone A, and the highest temperature $T^{maxB}$ which the reaction gas mixture has within temperature zone B is $\geq 0$°C,

wherein the transition from temperature zone A to temperature zone B in fixed catalyst bed does not coincide with a transition from one fixed catalyst bed zone to another fixed catalyst bed zone.

2. The process according to claim 1, wherein $T^{maxA} - T^{maxB}$ is $\geq 3$°C and $\leq 60$ °C.

3. The process according to claim 1, wherein $T^{maxA} - T^{maxB}$ is $\geq 5$ °C and $\leq 40$ °C.

4. The process according to any of claims 1 to 3, wherein the acrolein hourly space velocity on the fixed catalyst bed is $\geq 70$ l (STP)/l·h and < 150 l (STP)/l·h.

5. The process according to any of claims 1 to 3, wherein the propene hourly space velocity on the fixed catalyst bed is $\geq 150$ 1 (STP)/l·h and $\leq 300$ 1 (STP)/l·h.

6. The process according to any of claims 1 to 5, wherein the temperature of reaction zone A is from 250 to 300°C.

7. The process according to any of claims 1 to 5, wherein the temperature of reaction zone A is from 260 to 280°C.

8. The process according to any of claims 1 to 7, wherein temperature zone A extends to an acrolein conversion of from 50 to 85 mol%.

9. The process according to any of claims 1 to 8, wherein temperature zone A extends to an acrolein conversion of from 60 to 85 mol%.

10. The process according to any of claims 1 to 9, wherein the $O_2$: acrolein ratio in the starting reaction gas mixture is $\geq 1$.

11. The process according to any of claims 1 to 10, wherein the chemical composition of the active composition used is unchanged over the entire fixed catalyst bed.

12. The process according to any of claims 1 to 11, wherein the entire fixed catalyst bed comprises not more than 4 fixed catalyst bed zones.

13. The process according to any of claims 1 to 12, wherein, when the active composition is uniform over the entire fixed catalyst bed, the volume-specific active composition in the flow direction of the reaction gas mixture increases by at least 5% by weight at the transition from one fixed catalyst bed zone to another fixed catalyst bed zone.

14. The process according to any of claims 1 to 12, wherein, when the active composition is uniform over the entire fixed catalyst bed, the volume-specific active composition in the flow direction of the reaction gas mixture increases by at least 10% by weight at the transition from one fixed catalyst bed zone to another fixed catalyst bed zone.

15. The process according to any of claims 1 to 14, wherein, when the active composition is uniform over the entire fixed catalyst bed, the difference in the volume-specific active composition between the fixed catalyst bed zone having the lowest volume-specific activity and the fixed catalyst bed zone having the highest volume-specific activity is not more than 40% by weight.

16. The process according to any of claims 1 to 15, wherein the last fixed catalyst bed zone in the flow direction of the reaction gas mixture is undiluted and consists only of shaped catalyst bodies.

17. The process according to any of claims 1 to 16, wherein the fixed catalyst bed zone having the highest volume-specific activity extends into temperature zone A.

18. The process according to any of claims 1 to 16, wherein the fixed catalyst bed consists of four fixed catalyst bed zones and the fixed catalyst bed zone with the second highest volume-specific activity extends both into temperature zone A and into temperature zone B.

19. The process according to any of claims 1 to 18, wherein there is no transition from one fixed catalyst bed zone to another fixed catalyst bed in the fixed catalyst bed within the range of $X \pm L \cdot \dfrac{4}{100}$ where L is the length of the fixed catalyst bed and X is the point within the fixed catalyst bed of transition from temperature zone A to temperature zone B.

**Revendications**

1. Procédé d'oxydation partielle en phase gazeuse catalysée de manière hétérogène d'acroléine pour donner de l'acide acrylique, dans lequel un mélange initial de gaz réactionnel contenant de l'acroléine, de l'oxygène moléculaire et au moins un gaz inerte, l'oxygène moléculaire et l'acroléine étant contenus dans un rapport moléculaire $O_2$: $C_3H_4O \geq 0,5$, est conduit en une étape de réaction sur un remplissage de catalyseur à lit fixe dont la masse active est au moins un oxyde multimétallique contenant les éléments molybdène et vanadium, sous réserve que

   - le remplissage de catalyseur à lit fixe soit disposé dans deux zones de température A, B spatialement successives,
   - aussi bien la température de la zone de température A que la température de la zone de température B soient une température dans la plage de 230 à 320˚C,
   - le remplissage de catalyseur à lit fixe soit constitué d'au moins deux zones de remplissage de catalyseur à lit fixe spatialement successives, l'activité volumique spécifique étant essentiellement constante à l'intérieur d'une zone de remplissage de catalyseur à lit fixe et augmentant de manière inconstante dans le sens de l'écoulement du mélange de gaz réactionnel lors du passage d'une zone de remplissage de catalyseur à lit fixe à une autre zone de remplissage de catalyseur à lit fixe,
   - la zone de température A s'étende jusqu'à une conversion de l'acroléine de 45 à 85 % en moles,
   - lors du passage unique du mélange initial de gaz réactionnel à travers la totalité du remplissage de catalyseur à lit fixe, la conversion de l'acroléine soit $\geq$ 90 % en moles et la sélectivité de la formation d'acide acrylique, par rapport à l'acroléine convertie, soit $\geq$ 90 % en moles,
   - l'ordre chronologique dans lequel le mélange de gaz réactionnel traverse les zones de température A, B corresponde à l'ordre alphabétique des zones de température,
   - la charge du remplissage de catalyseur à lit fixe avec l'acroléine contenue dans le mélange initial de gaz réactionnel soit $\geq$ 70 Nl d'acroléine/l de remplissage de catalyseur à lit fixe • h, et
   - la différence $T^{maxA}$ - $T^{maxB}$, formée à partir de la température la plus élevée $T^{maxA}$ présentée par le mélange de gaz réactionnel à l'intérieur de la zone de température A et de la température la plus élevée $T^{maxB}$ présentée par le mélange de gaz réactionnel à l'intérieur de la zone de température B, soit $\geq$ 0˚C,

   **caractérisé en ce que** le passage de la zone de température A à la zone de température B dans le remplissage de catalyseur à lit fixe ne coïncide pas avec un passage d'une zone de remplissage de catalyseur à lit fixe à une autre zone de remplissage de catalyseur à lit fixe.

**2.** Procédé selon la revendication 1, **caractérisé en ce que** $T^{maxA} - T^{maxB}$ est $\geq 3°C$ et $\leq 60°C$.

**3.** Procédé selon la revendication 1, **caractérisé en ce que** $T^{maxA} - T^{maxB}$ est $\geq 5°C$ et $\leq 40°C$.

**4.** Procédé selon l'une quelconque des revendications 1 à 3, **caractérisé en ce que** la charge en acroléine du remplissage de catalyseur à lit fixe est $\geq 70$ N1/1 • h et $< 150$ NI/l • h.

**5.** Procédé selon l'une quelconque des revendications 1 à 3, **caractérisé en ce que** la charge en acroléine du remplissage de catalyseur à lit fixe est $\geq 150$ NI/l • h et $\leq 300$ NI/l • h.

**6.** Procédé selon l'une quelconque des revendications 1 à 5, **caractérisé en ce que** la température de la zone de réaction A est de 250 à 300°C.

**7.** Procédé selon l'une quelconque des revendications 1 à 5, **caractérisé en ce que** la température de la zone de réaction A est de 260 à 280°C.

**8.** Procédé selon l'une quelconque des revendications 1 à 7, **caractérisé en ce que** la zone de température A s'étend jusqu'à une conversion de l'acroléine de 50 à 85 % en moles.

**9.** Procédé selon l'une quelconque des revendications 1 à 8, **caractérisé en ce que** la zone de température A s'étend jusqu'à une conversion de l'acroléine de 60 à 85 % en moles.

**10.** Procédé selon l'une quelconque des revendications 1 à 9, **caractérisé en ce que** le rapport $O_2$: acroléine dans le mélange initial de gaz réactionnel est $\geq 1$.

**11.** Procédé selon l'une quelconque des revendications 1 à 10, **caractérisé en ce que** la composition chimique de la masse active utilisée est inchangée pour la totalité du remplissage de catalyseur à lit fixe.

**12.** Procédé selon l'une quelconque des revendications 1 à 11, **caractérisé en ce que** la totalité du remplissage de catalyseur à lit fixe ne comporte pas plus de quatre zones de remplissage de catalyseur à lit fixe.

**13.** Procédé selon l'une quelconque des revendications 1 à 12, **caractérisé en ce que**, dans le cas d'une masse active homogène pour la totalité du remplissage de catalyseur à lit fixe, la masse active volumique spécifique augmente d'au moins 5 % en poids, lors du passage d'une zone de remplissage de catalyseur à lit fixe à une autre zone de remplissage de catalyseur à lit fixe dans le sens de l'écoulement du mélange de gaz réactionnel.

**14.** Procédé selon l'une quelconque des revendications 1 à 12, **caractérisé en ce que**, dans le cas d'une masse active homogène pour la totalité du remplissage de catalyseur à lit fixe, la masse active volumique spécifique augmente d'au moins 10 % en poids, lors du passage d'une zone de remplissage de catalyseur à lit fixe à une autre zone de remplissage de catalyseur à lit fixe dans le sens de l'écoulement du mélange de gaz réactionnel.

**15.** Procédé selon l'une quelconque des revendications 1 à 14, **caractérisé en ce que**, dans le cas d'une masse active homogène pour la totalité du remplissage de catalyseur à lit fixe, la différence de masse active volumique spécifique des zones de remplissage de catalyseur à lit fixe ayant l'activité volumique spécifique la plus faible et des zones de remplissage de catalyseur à lit fixe ayant l'activité volumique spécifique la plus élevée n'est pas supérieure à 40 % en poids.

**16.** Procédé selon l'une quelconque des revendications 1 à 15, **caractérisé en ce que** la dernière zone de remplissage de catalyseur à lit fixe dans le sens de l'écoulement du mélange de gaz réactionnel est non diluée et ne se compose que de corps moulés de catalyseur.

**17.** Procédé selon l'une quelconque des revendications 1 à 16, **caractérisé en ce que** la zone de remplissage de catalyseur à lit fixe ayant l'activité volumique spécifique la plus élevée fait saillie jusque dans la zone de température A.

**18.** Procédé selon l'une quelconque des revendications 1 à 16, **caractérisé en ce que** le remplissage de catalyseur à lit fixe est constitué de quatre zones de remplissage de catalyseur à lit fixe et la zone de remplissage de catalyseur à lit fixe ayant la deuxième activité volumique spécifique la plus élevée fait saillie aussi bien dans la zone de température A que dans la zone de température B.

**19.** Procédé selon l'une quelconque des revendications 1 à 18, **caractérisé en ce que**, dans le remplissage de catalyseur à lit fixe, aucun passage d'une zone de remplissage de catalyseur à lit fixe à un autre remplissage de catalyseur à lit fixe ne se trouve dans la plage de $X \pm L \bullet \dfrac{4}{100}$ , L étant la longueur du remplissage de catalyseur à lit fixe et X étant le point à l'intérieur du remplissage de catalyseur à lit fixe où survient le passage de la zone de température A à la zone de température B.